(19) Europäisches Patentamt
European Patent Office
Office européen des brevets

(11) **EP 2 702 395 B1**

(12) **FASCICULE DE BREVET EUROPEEN**

(45) Date de publication et mention
de la délivrance du brevet:
**15.04.2015 Bulletin 2015/16**

(51) Int Cl.:
***G01N 21/65*** *(2006.01)*    ***G01N 21/35*** *(2014.01)*

(21) Numéro de dépôt: **12705694.3**

(86) Numéro de dépôt international:
**PCT/EP2012/053207**

(22) Date de dépôt: **24.02.2012**

(87) Numéro de publication internationale:
**WO 2012/116937 (07.09.2012 Gazette 2012/36)**

(54) **MÉTHODE DE DÉTERMINATION DE LA QUANTITÉ D'AU MOINS CERTAINS ACIDES GRAS CONTENUS DANS DIFFÉRENTES MATIÈRES BIOLOGIQUES D'UN MÊME ANIMAL ÉLEVÉ POUR SA PRODUCTION DE VIANDE**

VERFAHREN ZUR BESTIMMUNG DER MENGE VON ZUMINDEST EINIGEN FETTSÄUREN IN VERSCHIEDENEN BIOLOGISCHEN MATERIALIEN AUS EINEM FÜR DIE FLEISCHPRODUKTION GEZÜCHTETEN TIERES

METHOD FOR DETERMINING THE AMOUNT OF AT LEAST SOME FATTY ACIDS CONTAINED IN VARIOUS BIOLOGICAL MATERIALS FROM A SINGLE ANIMAL RAISED FOR MEAT PRODUCTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.03.2011 FR 1151737**

(43) Date de publication de la demande:
**05.03.2014 Bulletin 2014/10**

(73) Titulaire: **Valorex**
**35210 Combourtille (FR)**

(72) Inventeurs:
• **CHESNEAU, Guillaume**
**F-35133 Luitre (FR)**
• **WEILL, Pierre**
**F-35770 Vern Sur Seiche (FR)**

(74) Mandataire: **Regimbeau**
**Espace Performance Bâtiment K**
**35769 St-Grégoire Cedex (FR)**

(56) Documents cités:
**US-A1- 2005 250 212**

• **FERNÁNDEZ-CABANÁS V. M. ET AL: "Rapid determination of the fatty acid profile in pork dry-cured sausages by NIR spectroscopy", FOOD CHEMISTRY, ELSEVIER LTD, NL, vol. 124, no. 1, 1 janvier 2011 (2011-01-01), pages 373-378, XP027183509, ISSN: 0308-8146 [extrait le 2010-06-13]**
• **OLSEN E.F. ET AL: "Quantitative determination of saturated-, monounsaturated- and polyunsaturated fatty acids in pork adipose tissue with non-destructive Raman spectroscopy", MEAT SCIENCE, ELSEVIER SCIENCE, GB, vol. 76, no. 4, 27 avril 2007 (2007-04-27) , pages 628-634, XP022050639, ISSN: 0309-1740, DOI: 10.1016/J.MEATSCI. 2007.02.004**
• **GONZÁLEZ-MARTÍN I. ET AL: "On-line determination of fatty acid composition in intramuscular fat of Iberian pork loin by NIRs with a remote reflectance fibre optic probe", MEAT SCIENCE, ELSEVIER SCIENCE, GB, vol. 69, no. 2, 1 février 2005 (2005-02-01), pages 243-248, XP025283314, ISSN: 0309-1740, DOI: 10.1016/J.MEATSCI.2004.07.003 [extrait le 2005-02-01]**

- **FLATTEN A ET AL: "Determination of C22:5 and C22:6 marine fatty acids in pork fat with Fourier transform mid-infrared spectroscopy", MEAT SCIENCE, ELSEVIER SCIENCE, GB, vol. 69, no. 3, 1 mars 2005 (2005-03-01), pages 433-440, XP004689875, ISSN: 0309-1740, DOI: 10.1016/J.MEATSCI.2004.10.002**

- **AFSETH N K ET AL: "The potential of Raman spectroscopy for characterisation of the fatty acid unsaturation of salmon", ANALYTICA CHIMICA ACTA, ELSEVIER, AMSTERDAM, NL, vol. 572, no. 1, 14 juillet 2006 (2006-07-14), pages 85-92, XP025047676, ISSN: 0003-2670, DOI: 10.1016/J.ACA.2006.05.013 [extrait le 2006-07-14]**

**Description**

L'invention concerne une méthode de détermination de la quantité de certains acides gras contenus dans différentes matières biologiques d'un même animal élevé pour sa production de viande

La teneur en matière grasse (ou lipide) de chacun des produits que nous consommons fait partie des informations courantes figurant sur les étiquettes des conditionnements des aliments.

Ces lipides sont depuis longtemps rejetés en raison de leur forte contribution énergétique dans les régimes, les acides gras saturés en tête.

Depuis une dizaine d'année les scientifiques multiplient les preuves de ne pas rejeter les acides gras. Pour les nutritionnistes, il n'y a pas de bons et de mauvais acides gras dans les régimes de l'homme, mais seulement des acides gras en excès ou en déficit provoquant de nombreux déséquilibres physiologiques, qui contribuent à la génèse de nombreuses maladies dites « de civilisation » (maladie cardiovasculaire, diabète, obésité etc...)

Tous les guides nutritionnels s'accordent désormais à recommander :

a. une hausse de la consommation d'acide oléique (C18:1n-9),
b. une hausse de la consommation d'oméga 3 sous forme d'acide alpha-linolénique (C18:3n-3), d'EPA (C20:5 n-3) et de DHA (C22:6 n-3),
c. une limitation de la consommation d'acide palmitique (C16:0),
d. une limitation de la consommation d'acide linoléique (C18:2n-6),
e. une diminution du rapport C18:2n-6/C18:3n-3.

Ces dix dernieres années ont été riches en études cherchant à optimiser la composition nutritionnelle des acides gras des produits consommés par l'homme pour améliorer sa santé, en suivant ces recommandations. Ainsi, les modes de production des animaux et la composition en acides gras de leurs lipides sont passés au crible et des méthodes de détermination des acides gras se sont développées.

Pour ce qui concerne les animaux, leur alimentation joue un rôle majeur (supérieur à la génétique et autres facteurs d'élevage) dans la détermination de la composition en acides gras de leurs produits.

Ainsi, pour améliorer le profil d'acides gras d'une viande, d'un lait, d'un oeuf, il convient de bien choisir les matières premières constitutives de la ration alimentaire. Chez l'animal monogastrique, le profil lipidique de la viande est le reflet direct de son alimentation.

Chez le ruminant, ce lien existe aussi mais est « brouillé » par la fonction de bio hydrogénation du rumen, ce qui rend la corrélation plus délicate et tributaire des interactions digestives au sein du rumen.

Et au-delà de l'intérêt de connaître la composition en acides gras des produits consommés par l'homme pour la santé de ce dernier, il est également très intéressant de tenir compte de la composition en acides gras des matières biologiques constitutives des animaux pour caractériser la santé de ces animaux.

En effet, par la reconnaissance de certains acides gras ou ratio d'acides gras en des quantités connues, il est tout à fait possible de porter un diagnostic sur les déséquilibres nutritionnels d'une ration alimentaire et, par voie de conséquence, sur les désordres métaboliques encourus sur le moyen et le long terme.

Ainsi, les acides gras C18 :2 n-6 et C18 :3 n-3 ne peuvent avoir qu'une origine exogène (c'est-à-dire alimentaire), car aucun animal ne peut les synthétiser. Ces acides gras indispensables sont aussi dits essentiels car ils sont des vecteurs très importants de l'équilibre des systèmes hormonal, immunitaire, inflammatoire etc... et leurs dérivés oxygénés sont le plus souvent antagonistes, ce qui a amené les nutritionnistes à recommander un ratio C18 :2 n-6 / C18 :3 n-3 pour mieux équilibrer les grandes fonctions métaboliques des organismes, qu'il s'agisse de l'homme ou des animaux.

C'est pourquoi il devient intéressant de contrôler sur l'animal vivant, via l'analyse de son sang, de sa peau, de ses poils ou tout autre tissu biologique, l'équilibre de ce ratio (par exemple) lequel est fatalement corrélé à l'équilibre de ce ratio dans la ration alimentaire qui n'est, quant à lui, pas toujours connu.

Ceci est vrai pour des acides gras exclusivement d'origine exogène, mais peut tout à fait l'être pour des acides gras d'origine endogène (ou mixte), puisque leur synthèse est modulée par de nombreux équilibres d'une ration alimentaire : apports en énergie (sous forme de sucres, d'amidon, de lipides ou de glucides complexes), en protéine, en fibres, en minéraux, en vitamines.

En fonction du niveau énergétique d'une ration dépendra le niveau de synthèse et de lipolyse des acides gras du tissu adipeux, et donc le profil en acides gras de celui-ci sera modifié.

A la différence des huiles végétales, qui contiennent une vingtaine d'acides gras majeurs différents, les matières grasses animales, particulièrement issues de ruminants, sont composées d'un très grand nombre d'acides gras différents.

On connaît près de 400 acides gras différents. Les proportions relatives de ces acides gras sont extrêmement variables en fonction de nombreux paramètres : la race, l'individu, la saison, l'âge, et surtout l'alimentation.

Les acides gras sont classiquement regroupés en familles dont voici une liste illustrée de quelques exemples d'acides gras :

a. Les acides gras saturés à chaîne courte (C4:0, C6:0, C8:0, C10:0) ;
b. Les acides gras saturés à chaîne moyenne : acide laurique (C12:0) et acide myristique (C14:0);
c. Les acides gras saturés à chaîne longue : acide palmitique (C16 :0) et acide stéarique (C18:0) ;
d. Les acides gras monoinsaturés cis ou trans : acide oléique (C18:1cis9), acide vaccénique (C18:1trans11);
e. Les acides gras conjugués : acide ruménique (C18:2cis9 trans11);
f. Les acides gras impairs ramifiés ;
g. Les acides gras polyinsaturés de la famille Oméga 3: acide alpha-linolénique (C18:3n-3), acide eicosapentaénoïque (C20:5n-3), acide docosahexaénoïque (C22:6n-3);
h. des acides gras polyinsaturés de la famille Oméga 6: acide linoléique (C18:2n-6), acide arachidonique (C20:4 n-6).

La grande variété et la grande dispersion de composition des acides gras des produits animaux, d'une part, et l'importance quantitative de la consommation de matières grasses animales issues des viandes et produits dérivés de leur production, d'autre part, rendent très importants l'évaluation de la qualité nutritionnelle des lipides des produits d'animaux élevés pour la production de viande.

Les filières d'élevage s'orientent donc vers une amélioration de la qualité nutritionnelle des lipides des viandes et produits dérivés de leur production. Quant aux élevages en tant que tels, ils recherchent des outils de diagnostic de l'équilibre des rations alimentaires distribuées aux animaux pour optimiser la rentabilité économique de leur production par des meilleures performances et une meilleure santé des animaux.

Compte tenu de ce double engouement visant le producteur et le consommateur, il semble donc tout à fait intéressant de pouvoir évaluer de façon rapide, fiable et à moindre coût, la composition des lipides des viandes et produits dérivés de leur production sur l'animal mort, ainsi que celle des matières biologiques accessibles sur l'animal vivant (par analyse directe ou par prélèvement) afin de prédire, c'est à dire prévoir, le profil d'acides gras obtenu dans les viandes et produits dérivés. En effet, par la connaissance anticipée de la composition du sang, de la peau et du poil à quelques heures, jours ou mois de l'abattage de l'animal, pour une ration alimentaire donnée, il est envisageable de prédire la qualité des lipides des viandes et produits dérivés de leur production, dans le cadre d'une alimentation stable dans le temps. Ainsi, par exemple, si la quantité d'Oméga 3 diagnostiquée dans la viande par l'analyse du poil est insuffisante pour répondre à un objectif qualitatif selon un cahier de charges, alors un conseil de changement de la ration de l'animal peut être envisagé afin d'atteindre le but recherché

Ainsi, la présente invention se rapporte à une méthode de détermination de la quantité d'au moins certains acides gras contenus dans différentes matières biologiques d'un même animal élevé pour sa production de viande, à partir d'une base de données dans laquelle on a déterminé préalablement, pour une matière de référence provenant du même type d'animal, les compositions ou profils en acides gras, déterminées par chromatographie en phase gazeuse, puis mesuré et enregistré les spectres d'absorption Infrarouge ou de diffusion Raman correspondants, méthode comprenant les étapes suivantes :

a/ on détermine, par un modèle mathématique, pour cette matière de référence, une équation de calibration correspondant à la détermination, par une méthode spectroscopique, d'un acide gras ou d'une somme d'acides gras ;
b/ on valide l'emploi de ladite équation de calibration de l'étape précédente, en vue de son utilisation avec de nouveaux échantillons à analyser, dès lors que pour une série d'échantillons dits « de validation », de même nature que la matière biologique de référence, le coefficient de corrélation $r^2$ entre la teneur en l'acide gras considéré ou la somme d'acides gras obtenue par chromatographie en phase gazeuse et par spectroscopie, est au moins égal à 0.7 ;
c/ on soumet un nouvel échantillon à analyser, de même nature que la matière de référence, au rayonnement lumineux pour en obtenir le spectre d'absorption, et on utilise l'équation de calibration précédemment validée pour en déduire le profil, c'est à dire la teneur en acides gras dits « majeurs », à savoir ceux qui présentent un coefficient de corrélation $r^2$ au moins égal à 0.7 ,
caractérisé par le fait qu'il comprend également au moins l'une des étapes suivantes :
d/ on détermine le profil, c'est à dire la teneur en acides gras dits « mineurs », c'est à dire tous ceux dont le coefficient $r^2$ est inférieur à 0.7, à partir du profil en acides gras « majeurs » tel que déterminé à l'étape précédente, au moyen d'équations de prédiction statistiques présentant un coefficient de corrélation $r^2$ au moins supérieur au coefficient de corrélation $r^2$ déterminé entre les valeurs en acides gras dits « mineurs », mesuré par spectroscopie et les valeurs obtenues par chromatographie en phase gazeuse ou au moyen d'équations de prédiction statistiques présentant un coefficient de corrélation au moins égal à 0.7, obtenu avec la méthode de chromatographie en phase gazeuse ;
e/ on détermine la teneur en acides gras d'au moins une autre matière du même animal, à partir des profils obtenus aux étapes c/ et/ou d/, au moyen d'équations de prédiction statistiques présentant un coefficient de corrélation au moins égal à 0.7, obtenu avec la méthode de chromatographie en phase gazeuse.

Préférentiellement, aux étapes b/, c/, d/ et e/, $r^2$ est au moins égal à 0.8, préférentiellement au moins égal à 0.9.

Par ailleurs, selon d'autres caractéristiques avantageuses et non limitatives :

- ladite matière biologique est liquide ;
- ladite matière biologique est du sang ;
- ladite matière biologique est solide ;
- ladite matière biologique est choisi parmi les poils, les écailles, les plumes, la peau, le gras, la viande, les abats ;
- ledit animal est choisi dans le groupe constitué des bovins, des ovins, de la volaille, des porcs, des lapins et des poissons.

L'état de la technique le plus proche de l'invention est divulgué dans l'article FERNANDEZ-CABANÁS V. M. ET AL: "Rapid determination of the fatty acid profile in pork dry-cured sausages by NIR spectroscopy", FOOD CHEMISTRY, ELSEVIER LTD, NL, vol. 124, no. 1, 1 janvier 2011 (2011-01-01).

D'autres caractéristiques et avantages de la présente invention apparaîtront à la lecture de la description qui va suivre.

I - Modèle de détermination de la composition en acides gras des lipides des viandes et produits dérivés de leur production

a. Introduction

La détermination du profil en acides gras des lipides des produits s'effectue aujourd'hui par la méthode de référence : la chromatographie en phase gazeuse (CPG). Elle permet de séparer des mélanges gazeux par suite d'équilibre entre une phase gazeuse mobile et une phase stationnaire. Chronologiquement, la méthode repose sur :

1 - une étape d'extraction de la matière grasse,
2 - une préparation des esters méthyliques d'acides gras,
3 - une analyse par chromatographie en phase gazeuse de ces esters méthyliques d'acides gras.

Toutefois, cette méthode est lente (une à trois semaines selon les laboratoires) et onéreuse (100 à 200€ l'analyse environ). Elle est un frein considérable pour les acteurs des filières animales qui voudraient multiplier leur recherche, leur développement produit ou encore exercer un contrôle de la qualité des viandes obtenues ou de l'équilibre d'une ration alimentaire.

C'est ainsi qu'on a songé à une méthode tout aussi intéressante sur le plan de la fiabilité mais beaucoup plus rapide et moins onéreuse : l'analyse par spectroscopie infrarouge ou raman, couplée à des équations de prédiction.

La méthode proposée réside en 3 étapes :

Les deux premières étapes consistent à obtenir un profil d'acides gras de façon rapide, fiable et le plus complet possible pour un tissu donné d'un même animal.

La troisième étape permet d'obtenir un profil d'acide gras de façon rapide, fiable et le plus complet possible pour de nombreux tissus du même animal, et potentiellement à des temps différents, dès lors que la ration alimentaire n'a pas changé.

b. Description de la méthode

Etape 1 : Détermination partielle de la composition lipidique (à partir des acides gras majeurs, présents en plus grande quantité)

- d'un ou plusieurs tissus maigre (viande) ou gras (gras de couverture, gras intermusculaires) d'un animal élevé puis abattu pour sa production de viande (porc, bovin, ovin, volaille, lapin, poisson etc...)
- d'un ou plusieurs tissus (peau, poil...) ou matières biologiques (sang, urine, fécès, lait...) d'un animal en croissance ou en engraissement.

par la méthode d'analyse rapide et éventuellement non invasive de spectroscopie, infrarouge ou Raman, à partir des équipements de mesure disponibles sur le marché ou bien ceux à créer pour chacune des applications spécifiques qu'il pourrait être nécessaire de réaliser (par exemple sur la ligne d'abattage des porcs, des boeufs, des volailles etc... ou sur leur épiderme) pour gagner en praticité, en robustesse, en coût, en réactivité...

Après la constitution d'une base de données importante et représentative d'échantillons pour lesquels d'une part, on a déterminé la composition du profil d'acides gras par la méthode de référence (CPG) et d'autre part, on a obtenu des spectres d'absorption lumineuse à partir d'un équipement de mesure adapté (mais évolutif), et obtenu une calibration Infrarouge ou Raman pour chaque acide gras considéré par la méthode de référence.

Chaque calibration est caractérisée par un degré de fiabilité. Plus les acides gras ou familles d'acides gras (ex : acides gras saturés) sont présents en grande quantité (acides gras majeurs ; par exemple, acide palmitique, acide oléique, acide stéarique etc...) plus cette fiabilité sera forte.

En revanche pour obtenir rapidement par spectroscopie et de façon fiable des acides gras en petite quantité (acides gras dits mineurs ; par exemple, l'acide α-linolénique, oméga 3), dans le but d'obtenir un profil d'acides gras complet, une deuxième étape est nécessaire.

Etape 2 : Détermination des acides gras mineurs par équation de prédiction mathématiques.

Les acides gras d'un même tissu sont parfois très bien corrélés entre eux.

Ainsi, à partir des acides gras majeurs obtenus par spectroscopie, Infrarouge ou Raman, il est possible de calculer par équation de prédiction (linéaire ou non), la quantité d'acides gras dits mineurs, présents en petite quantité. Dès lors que cette seconde méthode d'évaluation par calcul offre une meilleure fiabilité de résultat que la première, elle mérite d'être utilisée.

Une autre évaluation d'acides gras insuffisamment bien prédits directement par spectroscopie, Infrarouge ou Raman, peut également être obtenue par équation de prédiction mathématique en lien avec d'autres constituants que les acides gras (eau, matières grasses, protéines, collagène, sel, etc.) dès lors qu'une corrélation étroite aura permis d'établir une équation de prédiction fiable.

Compte tenu du nombre et de la variabilité de composition des tissus sur un même animal, une troisième étape est envisageable pour obtenir rapidement la composition en acides gras des différentes tissus, éventuellement avec un décalage dans le temps si la ration alimentaire de l'animal reste stable.

Etape 3 : Détermination de la composition en acides gras de différents tissus d'un même animal, ou de plusieurs animaux d'un même lot de production homogène à partir de la connaissance du profil d'acides gras (selon les étapes 1 et/ou 2) d'au moins un tissu du même animal ou d'un animal du même lot.

En effet, en établissant des corrélations fortes de composition en acides gras entre différents tissus permettant des prédictions fiables, il devient possible de connaître la teneur en un acide gras d'un tissu (ex : muscle maigre) par connaissance de ce même acide gras dans un autre tissu (gras de couverture).

De la même manière, pour des productions très homogènes et standardisées avec peu de variabilité de composition en acides gras d'un animal à l'autre (cas des animaux élevés au sein d'un même élevage et recevant la même alimentation jusqu'au même âge), il est possible de prédire la teneur en un acide gras d'un tissu d'un animal à partir de la teneur en ce même acide gras d'un tissu d'un autre animal du même lot.

Enfin, il est également possible de connaître par anticipation la composition d'un tissu (ex : viande) en un acide gras donné à un instant t (ex : abattage), à partir de la connaissance de la composition en un autre tissu (ex : sang) à un instant t-h (en heure), t-j (en jour) ou t-s (en semaine), à partir du moment où l'animal reçoit une ration alimentaire stable.

Ainsi, par l'utilisation de méthodes d'analyses rapides de détermination des acides gras des produits, combinée à l'application d'équations de prédiction entre acides gras (ou encore à partir d'autres constituants que les acides gras mais bien prédit par spectroscopie et bien corrélé à l'acide gras considéré), ou aussi entre pièces à un temps donné (gras de couverture, gras intermusculaires, muscles variés) ou entre temps décalés (sang, poil, peau), ou encore entre animaux d'un lot homogène de production, ou enfin entre le début et la fin d'un procédé de cuisson ou autre transformation, il devient possible :

- d'accéder facilement à des informations nutritionnelles importantes à savoir les taux d'acides saturés, mono-insaturés, poly-insaturés, palmitique (C16:0), oléique (C18:1n-9), linoléique (C18:2 n-6), alpha-linolénique (C18:3n-3), linoléiques conjugués (CLA) etc... pour chacun des produits alimentaires à considérer,
- d'instaurer un système de paiement des produits (lait, beurre, fromage, viande, jambon, charcuterie, oeuf etc...) selon leur qualité nutritionnelle en acides gras, et ainsi dynamiser la mise en marché de produits de qualité,
- de contrôler rapidement et efficacement les produits d'animaux élevés pour leur viande et améliorer ainsi la constitution de cahier des charges avec obligation de résultats et plan de contrôle analytique des produits entre les producteurs, abatteurs, transformateurs et distributeurs,
- d'accélérer le processus d'acquisition des connaissances par la multiplication possible de l'étude des facteurs d'amélioration de la qualité,
- de renforcer la création de filières pour la promotion de produits de qualité nutritionnelle supérieure.

- d'améliorer l'équilibre de la ration des animaux, la santé des animaux et la rentabilité économique des systèmes de production des animaux.

II - Exemples pratiques

Etape 1 : Détermination partielle du profil en acides gras par spectroscopie (exemple de l'infrarouge).

Cette étape vise à identifier les acides gras prédictibles de manière fiable et robuste par mesure infrarouge. Cette étape nécessite le développement de calibration sur l'ensemble des acides gras mesurés par CPG. Les meilleures calibrations par acides gras seront ensuite retenues et définiront la liste des acides gras prédictibles de manière fiables par mesure infrarouge.

Cette illustration repose sur des données acquises sur des côtes de porc par un appareil aux caractéristiques suivantes :

- le mode d'acquisition des spectres utilisé a été la transmission (le spectre Infrarouge traverse l'échantillon).
- la gamme spectrale utilisée s'étend de 780 à 2500nm.

Dans cette illustration, le développement des modèles mathématiques pour la détermination des calibrations a été réalisé à l'aide du logiciel "Unscrambler".

La méthodologie décrite ci-dessous est transposable à l'ensemble des tissus et des appareils spectroscopiques à considérer.

a) Acquisition des spectres infrarouge et des analyses de référence (profils en acides gras)

L'acquisition des mesures spectrales a été réalisée sur 70 échantillons broyés de côtes de porcs, lesquels ont reçu une alimentation différenciée sur la teneur en C18 :3 n-3.

En parallèle de ces mesures, les profils en acides gras de ces mêmes échantillons de tissus ayant servis pour l'acquisition des profils spectraux ont été déterminés par Chromatographie en Phase Gazeuse (méthode de référence).

b) Construction de la base de données.

Une base de données regroupant pour chaque échantillon les valeurs de chaque point composant son spectre IR ainsi que son profil en acides gras a été constituée, comme illustré ci-dessous

Structure de la base de données permettant la recherche par méthode statistique de relations entre profil spectral obtenu par IR et profil en acides gras obtenu par CPG.

| | | Longueur d'onde | | | | | Profils en acides gras | | | | |
|---|---|---|---|---|---|---|---|---|---|---|---|
| N° echantillon | Régime | 400 | 402 | 404 | ... | n | C10:0 | C12:0 | C18:3 n-3 | ... | C24:1 |
| 1 | A | | | | | | | | | | |
| 2 | A | | | | | | | | | | |
| 3 | B | | | | | | | | | | |
| 4 | B | | | | | | | | | | |
| .... | .... | | | | | | | | | | |
| n | n | | | | | | | | | | |

c/ Construction des calibrations

A partir de cette base de données, différentes méthodes statistiques sont utilisées afin d'identifier les acides gras qui sont prédictibles de façon fiable et précise, à partir du spectre infrarouge.

Dans cette illustration, l'ensemble des spectres est standardisé par la méthode MSC (Multiplicative Scatter Correction). Cette transformation permet de limiter la dispersion des spectres les uns par rapport aux autres. D'autres prétraitements mathématiques du signal infrarouge peuvent être utilisés. Le choix du pré-traitement (dérivé première, dérivée seconde, MSC,...) peut impacter sensiblement sur la qualité de la calibration. Il est donc nécessaire par l'intermédiaire de logiciels dédiés de choisir le pré-traitement mathématique optimal.

Pour chaque point du spectre I.R., un coefficient de régression pour chaque acide gras est calculé par la méthode de la Régression des Moindres Carrés Partiels (PLS1).

Chaque point du spectre IR est multiplié par le coefficient de régression correspondant. La somme de ces produits correspond alors à la valeur prédite de l'acide gras considéré en fonction du spectre infrarouge.

<u>d) Validation interne</u>

L'évaluation du modèle est réalisée par la méthode dite de « validation croisée » (cross-validation).

Dans cette méthode, le jeu de données des échantillons servant à la calibration est divisé en n sous-ensemble. La calibration est réalisée avec les n-1 sous ensembles, le dernier sous-ensemble servant comme jeu de données pour la validation. Le processus est répété autant de fois qu'il y a de sous-ensemble. Cette méthode permet de valider le modèle et de tester la capacité prédictive du modèle.

<u>e)Evaluation de la qualité de la calibration</u>

L'évaluation de la qualité des prédictions est effectuée à partir du coefficient de corrélation de la régression ($R^2$) et de l'erreur quadratique moyenne de la prédiction (RMSEP).

Le Tableau 1 suivant synthétise les critères de qualité nécessaire à l'évaluation de la qualité du modèle de prédiction. Il est ainsi possible de définir de manière fiable l'ensemble des acides gras présents dans le tableau ci-dessous. Seuls les rapport n-6/n-3 semblent, à ce stade de l'étude, potentiellement difficile à prédire. Cependant une base de données plus large, avec une bonne représentativité des modes de production, peut être constituée pour vérifier ces niveaux de fiabilité.

Tableau 1: Critères de qualité des prédictions obtenues sur les côtes de porc sur une liste restreinte d'acides gras, et à partir d'une base de données encore limitée.

|  | Nb spectres dans la base de données | Teneurs Min. | Teneurs Max. | RMSEP | $R^2$ |
|---|---|---|---|---|---|
| AGS | 138 | 33,7 | 46,7 | 0,63 | 92,13 |
| AGMI | 134 | 40,2 | 49,1 | 0,88 | 83,76 |
| AGPIn-6 | 146 | 6,4 | 15,7 | 0,98 | 80,53 |
| AGPIn-3 | 136 | 1,2 | 4,8 | 0,35 | 81,38 |
| C16 :0 | 140 | 20,7 | 27,4 | 0,46 | 83,5 |
| ALA (C18:3n-3) | 133 | 1 | 3,8 | 0,29 | 79,04 |
| LA (C18 :2n-6) | 144 | 5,8 | 14,4 | 0,85 | 83,75 |
| n-6/n-3 | 138 | 2,2 | 5,9 | 0,53 | 49,06 |
| AGS/n-3 | 136 | 7,9 | 22,2 | 1,63 | 80,04 |
| AGS = acides gras saturés ; AGMI = acides gras monoinsaturés ; AGPI = acides gras polyinsaturés RMSEP = erreur quadratique moyenne de la prédiction | | | | | |

<u>f) Validation externe</u>

La robustesse, c'est-à-dire la validité des équations (ou du modèle de prédiction) déterminées précédemment est ensuite testée en utilisant des échantillons n'ayant pas servi au développement de la calibration. Il s'agit de déterminer la différence entre les valeurs prédites par le modèle sur ces échantillons et les valeurs réelles de l'acide gras considéré et mesurés par CPG. Plus l'écart est faible, meilleure est la robustesse du modèle. Cette étape ne peut être effectuée qu'avec des calibrations déjà développées à partir d'une base de données suffisamment fournie et représentative des pratiques du terrain.

<u>g) Conclusion</u>

Au terme de cette première étape, il est possible de lister les acides gras qu'il est possible de quantifier de manière fiable, précise et robuste à partir d'une mesure infrarouge d'un type d'échantillon, de tissu ou de viande.

Dans le cas de la côte de porc, les acides gras pouvant être listés comme étant mesurables par une acquisition du spectre infrarouge de l'échantillon, c'est-à-dire pour lesquels le coefficient de corrélation est supérieur à 0,7 préférentiellement 0,8, sont : la somme des acides gras saturés, et mono-insaturés, le C16 :0, la somme des oméga 6 et des oméga 3, le C18 :2 n-6 et le C18:3 n-3.

Cette liste n'est pas exhaustive et pourra être modifiées (en addition ou en soustraction d'acides gras) au fur et à mesure de l'incrémentation de nouvelles données qui permettraient d'étendre la base de données pour obtenir une meilleure représentativité de la production considérée.

Pour les autres acides gras non prévisibles par infrarouge, c'est à dire ceux dont le coefficient de corrélation ne dépasse par 0,7, ou préférentiellement 0,8, il est possible de déterminer leurs teneurs à partir d'étude statistique de corrélation et de prédiction avec les acides gras préalablement déterminés par infrarouge.

ETAPE 2: Détermination des acides gras mineurs par équation de prédiction mathématiques au sein d'un même tissu.

Il s'agit dans cette étape, à partir de la base de données ayant servi à constituer les calibrations spectroscopiques et/ou d'une base de données idéalement représentative de la production considérée, de mettre en évidence des corrélations statistiques et d'établir des équations de prédiction des acides gras non mesurables par spectroscopie par les teneurs des acides gras mesurables par spectroscopie.

Les résultats présentés dans les tableaux 2 à 5 sont obtenus sur des côtes de porc. Les tableaux 2 et 3 présentent respectivement les corrélations et les prédictions entre acides gras exprimés en % des acides gras totaux, les tableaux 4 et 5, entre acides gras exprimés en mg pour 100g de tissu.

A titre d'exemple :

Si la méthode spectroscopique permet de déterminer les AGMI mais pas le C18 :1 n-9, alors, si la corrélation entre AGMI et C18 :1 n-9 est hautement significative (probabilité $P < 0.001$), il devient possible de déterminer le C18 :1 n-9 par une équation de prédiction.

Si la mesure des acides gras est exprimée en % des acides gras totaux, alors l'équation, issue du tableau 2, est :

$$C18 :1 \; n\text{-}9 = 1.02 * AGMI - 5.64$$

D'une manière plus élaborée, il est également envisageable de prédire un acide gras par une équation à plusieurs inconnues. Par exemple, il est tout à fait possible de déterminer la somme des acides gras oméga 3 à longues chaînes (AGPI LC n-3), c'est-à-dire ceux dont le nombre de carbone est supérieur à 18, par l'équation suivante :

$$AGPI \; LC \; n\text{-}3 = AGPI \; n\text{-}3 - C18 :3 \; n\text{-}3$$

Les résultats présentés dans les tableaux 6 à 9 sont obtenus sur des muscles de bovins. Les tableaux 6 et 7 présentent respectivement les corrélations et les prédictions entre acides gras exprimés en % des acides gras totaux, les tableaux 8 et 9, entre des acides gras exprimés en mg pour 100g de produit.

D'autres tableaux pourraient ainsi être constitués pour d'autres tissus, et d'autres espèces animales.

Par ailleurs, si la méthode spectroscopique permet de déterminer certains acides gras (ou famille d'acide gras) d'un tissu donné, mais ne permet pas déterminer ces mêmes acides gras sur d'autres tissus du même animal (qui par exemple, en contiennent moins en valeur absolue), la méthode propose une 3ème étape.

ETAPE 3 : Prédiction des teneurs en acides gras de différents tissus à partir du profil en acides gras d'un seul tissu.

Comme précédemment, il s'agit à cette étape d'utiliser des équations issues des meilleures corrélations permettant de déterminer un acides gras donné (ou famille d'acide gras) d'un ou plusieurs tissus à partir de sa teneur dans un autre tissu, à l'issue de l'étape 1 ou de l'étape 2.

En utilisant des bases de données constituées des profils en acides gras (déterminés par CPG) de différents tissus d'un même animal, des corrélations entre tissus pour chaque acides gras ont été réalisées.

Les résultats présentés dans les tableaux 10 et 11 sont obtenus sur le C18 :3 n-3 de différents tissus respectivement du porc et du bovin.

A titre d'exemple, à l'issue de l'étape 1 :

Si le C18 :3 n-3 est déterminé par spectroscopie sur la bardière du porc, alors il est possible de déterminer le C18:3 du semi-membraneux (SM) du porc par l'équation suivante : % C18 :3 SM = 0.49 x %C18 :3 Bardière + 0.11

Si le C16 :0 est déterminé par spectroscopie sur le long dorsal du jeune bovin, alors il est possible de déterminer le C16 :0 de la bavette du jeune bovin par l'équation suivante : % C16 :0 Bavette = 4.59 + 0.82 x % C16 Long dorsal

Et de rajouter éventuellement la notion de temps :

Si le C18 :3 n-3 est déterminé par spectroscopie sur le plasma du porc au jour J, alors il est possible de déterminer le C18 :3 de la côte de porc à l'abattage (J+30) à condition d'une alimentation inchangée, par l'équation suivante :

$$\% \text{ C18 :3 côte porc} = 0.58 \times \%\text{C18 :3 Plasma} + 0.36$$

A titre d'exemple à l'issue de l'étape 2 :

Si le C16 :0 du long dorsal du jeune bovin est déterminé par spectroscopie en % des AG totaux, alors il est possible :

a) de déterminer le C18 :3 n-3 du long dorsal par l'équation suivante :

C18 :3 long dorsal = 3.25 - 0.1 x C16 :0 long dorsal

b) de déterminer le C18:3 n-3 de la bavette par l'équation suivante :

C18 :3 bavette = 0.22 + 0.62 x C18 :3 long dorsal

Grâce à la méthode de détermination proposée, il est possible de connaître rapidement la qualité nutritionnelle des lipides des viandes, et tout produit dérivé de sa production, pour toutes espèces animales, dès lors que des équations de prédiction fiables viennent compléter l'analyse spectroscopique réalisée sur au moins un tissu de l'animal, mort (gras de couverture, gras interne, inter- ou intra-musculaire...) ou vivant (sang, peau, poil...), et dont les acides gras qui viendraient à être prédits par équation le seraient plus précisément que par spectroscopie.

**Tableau 2: Exemple de matrice de corrélation ($r^2$) inter-acides gras (en % des acides gras totaux) obtenue sur la côte de porc (n=107). En italique, les relations significatives au seuil de 0.001%.**

| | C18:3n-3 | AGS | AGM | AGPI | n-6 | n-3 | C16:0 | C16:1n-7 | C18:1n-9c | C18:2n-6c | C20:4n-6 | C20:5n-3 | C22:5n-3 | C22:6n-3 |
|---|---|---|---|---|---|---|---|---|---|---|---|---|---|---|
| **C18:3n-3** | $r^2$=1.00 | *$r^2$=0.24* | *$r^2$=0.54* | *$r^2$=0.62* | $r^2$=0.05 | *$r^2$=0.94* | *$r^2$=0.45* | *$r^2$=0.46* | *$r^2$=0.52* | *$r^2$=0.08* | $r^2$=0.01 | *$r^2$=0.24* | *$r^2$=0.60* | $r^2$=0.01 |
| **AGS** | *$r^2$=0.24* | $r^2$=1.00 | *$r^2$=0.07* | *$r^2$=0.49* | $r^2$=0.05 | *$r^2$=0.33* | *$r^2$=0.70* | *$r^2$=0.11* | *$r^2$=0.11* | $r^2$=0.00 | *$r^2$=0.15* | *$r^2$=0.35* | *$r^2$=0.26* | *$r^2$=0.12* |
| **AGM** | *$r^2$=0.54* | *$r^2$=0.07* | $r^2$=1.00 | *$r^2$=0.77* | $r^2$=0.04 | *$r^2$=0.61* | *$r^2$=0.35* | *$r^2$=0.35* | *$r^2$=0.89* | $r^2$=0.00 | $r^2$=0.06 | *$r^2$=0.29* | *$r^2$=0.40* | *$r^2$=0.07* |
| **AGPI** | *$r^2$=0.62* | *$r^2$=0.49* | *$r^2$=0.77* | $r^2$=1.00 | *$r^2$=0.07* | *$r^2$=0.75* | *$r^2$=0.73* | *$r^2$=0.36* | *$r^2$=0.76* | $r^2$=0.00 | *$r^2$=0.14* | *$r^2$=0.48* | *$r^2$=0.52* | *$r^2$=0.13* |
| **n-6** | $r^2$=0.05 | $r^2$=0.05 | $r^2$=0.04 | *$r^2$=0.07* | $r^2$=1.00 | $r^2$=0.04 | $r^2$=0.00 | *$r^2$=0.08* | $r^2$=0.01 | *$r^2$=0.82* | *$r^2$=0.12* | $r^2$=0.00 | $r^2$=0.01 | $r^2$=0.02 |
| **n-3** | *$r^2$=0.94* | *$r^2$=0.33* | *$r^2$=0.61* | *$r^2$=0.75* | $r^2$=0.04 | $r^2$=1.00 | *$r^2$=0.62* | *$r^2$=0.55* | *$r^2$=0.66* | *$r^2$=0.13* | $r^2$=0.01 | *$r^2$=0.43* | *$r^2$=0.62* | $r^2$=0.04 |
| **C16:0** | *$r^2$=0.45* | *$r^2$=0.70* | *$r^2$=0.35* | *$r^2$=0.73* | $r^2$=0.00 | *$r^2$=0.62* | $r^2$=1.00 | *$r^2$=0.50* | *$r^2$=0.43* | $r^2$=0.04 | *$r^2$=0.19* | *$r^2$=0.51* | *$r^2$=0.40* | *$r^2$=0.16* |
| **C16:1n-7** | *$r^2$=0.46* | *$r^2$=0.11* | *$r^2$=0.35* | *$r^2$=0.36* | *$r^2$=0.08* | *$r^2$=0.55* | *$r^2$=0.50* | $r^2$=1.00 | *$r^2$=0.43* | *$r^2$=0.16* | $r^2$=0.00 | *$r^2$=0.23* | *$r^2$=0.22* | $r^2$=0.00 |
| **C18:1n-9c** | *$r^2$=0.52* | *$r^2$=0.11* | *$r^2$=0.89* | *$r^2$=0.76* | $r^2$=0.01 | *$r^2$=0.66* | *$r^2$=0.43* | *$r^2$=0.43* | $r^2$=1.00 | $r^2$=0.03 | *$r^2$=0.12* | *$r^2$=0.44* | *$r^2$=0.44* | *$r^2$=0.11* |
| **C18:2n-6c** | *$r^2$=0.08* | $r^2$=0.00 | $r^2$=0.00 | $r^2$=0.00 | *$r^2$=0.82* | *$r^2$=0.13* | $r^2$=0.04 | *$r^2$=0.16* | $r^2$=0.03 | $r^2$=1.00 | $r^2$=0.00 | *$r^2$=0.12* | $r^2$=0.05 | $r^2$=0.01 |
| C20:4n-6 | $r^2$=0.01 | *$r^2$=0.15* | $r^2$=0.06 | *$r^2$=0.14* | *$r^2$=0.12* | $r^2$=0.01 | *$r^2$=0.19* | $r^2$=0.00 | *$r^2$=0.12* | $r^2$=0.00 | $r^2$=1.00 | *$r^2$=0.33* | $r^2$=0.03 | *$r^2$=0.29* |
| **C20:5n-3** | *$r^2$=0.24* | *$r^2$=0.35* | *$r^2$=0.29* | *$r^2$=0.48* | $r^2$=0.00 | *$r^2$=0.43* | *$r^2$=0.51* | *$r^2$=0.23* | *$r^2$=0.44* | *$r^2$=0.12* | *$r^2$=0.33* | $r^2$=1.00 | *$r^2$=0.30* | *$r^2$=0.17* |
| **C22:5n-3** | *$r^2$=0.60* | *$r^2$=0.26* | *$r^2$=0.40* | *$r^2$=0.52* | $r^2$=0.01 | *$r^2$=0.62* | *$r^2$=0.40* | *$r^2$=0.22* | *$r^2$=0.44* | $r^2$=0.05 | $r^2$=0.03 | *$r^2$=0.30* | $r^2$=1.00 | *$r^2$=0.20* |
| C22:6n-3 | $r^2$=0.01 | *$r^2$=0.12* | *$r^2$=0.07* | *$r^2$=0.13* | $r^2$=0.02 | $r^2$=0.04 | *$r^2$=0.16* | $r^2$=0.00 | *$r^2$=0.11* | $r^2$=0.01 | *$r^2$=0.29* | *$r^2$=0.17* | *$r^2$=0.20* | $r^2$=1.00 |

EP 2 702 395 B1

Tableau 3 : Matrice d'équation de prédiction inter-acides gras (en % des acides gras totaux) obtenus sur la cote de porc (n=107).

Equation du type Y = aX+b. En italique, les relations significatives au seuil de 0.001%.

| Y \ X | C18:3n-3 | AGS | AGM | AGPI | n-6 | n-3 | C16:0 | C16:1n-7 | C18:1n-9c | C18:2n-6c | C20:4n-6 | C20:5n-3 | C22:5n-3 | C22:6n-3 |
|---|---|---|---|---|---|---|---|---|---|---|---|---|---|---|
| C18:3n-3 | | $Y=-0.63X+28.76$ | $Y=-0.63X+30.17$ | $Y=0.50X-5.43$ | $Y=-0.35X+8.07$ | $Y=0.71X+0.03$ | $Y=-0.99X+27.28$ | $Y=-3.71X+10.73$ | $Y=-0.57X+25.02$ | $Y=-0.43X+8.53$ | $Y=-0.49X+4.06$ | $Y=5.01X+2.53$ | $Y=17.74X-0.53$ | $Y=4.95X+3.53$ |
| AGS | $Y=-0.38X+41.12$ | | $Y=0.18X+32.14$ | $Y=-0.35X+45.99$ | $Y=-0.27X+42.99$ | $Y=-0.33X+41.41$ | $Y=0.97X+16.71$ | $Y=1.39X+37.07$ | $Y=0.21X+31.98$ | $Y=-0.01X+39.85$ | $Y=-2.05X+40.98$ | $Y=-4.69X+40.83$ | $Y=-9.16X+41.90$ | $Y=-15.69X+40.40$ |
| AGM | $Y=-0.86X+45.29$ | $Y=0.41X+25.94$ | | $Y=-0.65X+54.01$ | $Y=-0.35X+46.37$ | $Y=-0.67X+45.56$ | $Y=1.03X+17.74$ | $Y=3.76X+34.99$ | $Y=0.88X+9.38$ | $Y=-0.02X+42.32$ | $Y=-1.93X+43.27$ | $Y=-6.41X+43.62$ | $Y=-16.89X+46.13$ | $Y=-17.73X+42.86$ |
| AGPI | $Y=1.24X+13.59$ | $Y=-1.41X+74.06$ | $Y=-1.18X+67.86$ | | $Y=0.62X+10.64$ | $Y=1.00X+13.03$ | $Y=-1.99X+65.55$ | $Y=-5.16X+27.93$ | $Y=-1.08X+58.65$ | $Y=0.04X+17.82$ | $Y=3.98X+15.75$ | $Y=11.10X+15.55$ | $Y=26.05X+11.96$ | $Y=33.42X+16.74$ |
| n-6 | $Y=-0.15X+12.78$ | $Y=-0.20X+19.96$ | $Y=-0.11X+16.95$ | $Y=0.11X+10.18$ | | $Y=-0.10X+12.74$ | $Y=-0.06X+13.70$ | $Y=1.03X+10.27$ | $Y=-0.04X+13.68$ | $Y=0.89X+2.32$ | $Y=1.54X+11.23$ | $Y=-0.15X+12.24$ | $Y=-1.50X+12.56$ | $Y=5.00X+11.98$ |
| n-3 | $Y=1.31X+0.28$ | $Y=-1.00X+44.96$ | $Y=-0.90X+43.21$ | $Y=0.75X-8.43$ | $Y=-0.43X+10.49$ | | $Y=-1.58X+42.70$ | $Y=-5.49X+15.51$ | $Y=-0.87X+37.70$ | $Y=-0.73X+13.30$ | $Y=1.04X+4.55$ | $Y=9.11X+2.99$ | $Y=24.55X-0.71$ | $Y=15.65X+4.50$ |
| C16:0 | $Y=-0.45X+25.41$ | $Y=0.73X-5.09$ | $Y=0.34X+9.42$ | $Y=-0.37X+30.42$ | $Y=-0.07X+24.52$ | $Y=-0.39X+25.76$ | | $Y=2.60X+18.85$ | $Y=0.35X+10.61$ | $Y=0.21X+21.36$ | $Y=-1.98X+24.96$ | $Y=-4.93X+24.92$ | $Y=-9.76X+26.08$ | $Y=-15.86X+24.44$ |
| C16:1n-7 | $Y=-0.12X+2.35$ | $Y=0.08X-1.19$ | $Y=0.09X-2.00$ | $Y=-0.07X+3.16$ | $Y=0.08X+0.91$ | $Y=-0.10X+2.40$ | $Y=0.19X-2.67$ | | $Y=0.10X-1.67$ | $Y=0.11X+0.66$ | $Y=0.00X+1.88$ | $Y=-0.90X+2.10$ | $Y=-1.96X+2.35$ | $Y=-0.47X+1.90$ |
| C18:1n-9c | $Y=-0.91X+40.72$ | $Y=0.54X+15.69$ | $Y=1.02X-5.64$ | $Y=-0.70X+50.08$ | $Y=-0.14X+39.07$ | $Y=-0.76X+41.24$ | $Y=1.23X+8.01$ | $Y=4.52X+28.82$ | | $Y=0.33X+33.65$ | $Y=-3.00X+39.18$ | $Y=-8.58X+39.39$ | $Y=-19.24X+41.94$ | $Y=-24.99X+38.43$ |
| C18:2n-6c | $Y=-0.19X+11.87$ | $Y=-0.01X+11.57$ | $Y=-0.01X+11.47$ | $Y=0.01X+11.01$ | $Y=0.92X-0.07$ | $Y=-0.18X+12.07$ | $Y=0.21X+6.12$ | $Y=1.48X+8.36$ | $Y=0.09X+7.67$ | | $Y=-0.27X+11.31$ | $Y=-2.39X+11.72$ | $Y=-3.51X+11.99$ | $Y=-4.22X+11.33$ |
| C20:4n-6 | $Y=-0.01X+0.67$ | $Y=-0.07X+3.58$ | $Y=-0.03X+1.93$ | $Y=0.04X-0.02$ | $Y=0.08X-0.32$ | $Y=0.01X+0.56$ | $Y=-0.10X+2.90$ | $Y=0.00X+0.63$ | $Y=-0.04X+2.17$ | $Y=-0.01X+0.77$ | | $Y=0.87X+0.42$ | $Y=0.57X+0.49$ | $Y=4.67X+0.42$ |
| C20:5n-3 | $Y=0.05X+0.06$ | $Y=-0.07X+3.19$ | $Y=-0.04X+2.13$ | $Y=0.04X-0.54$ | $Y=0.00X+0.28$ | $Y=0.05X-0.00$ | $Y=-0.10X+2.71$ | $Y=-0.26X+0.72$ | $Y=-0.05X+2.17$ | $Y=-0.05X+0.81$ | $Y=0.38X+0.01$ | | $Y=1.23X-0.05$ | $Y=2.38X+0.14$ |
| C22:5n-3 | $Y=0.03X+0.11$ | $Y=-0.03X+1.38$ | $Y=-0.02X+1.23$ | $Y=0.02X-0.12$ | $Y=-0.01X+0.32$ | $Y=0.03X+0.11$ | $Y=-0.04X+1.21$ | $Y=-0.11X+0.45$ | $Y=-0.02X+1.09$ | $Y=-0.01X+0.41$ | $Y=0.05X+0.21$ | $Y=0.24X+0.18$ | | $Y=1.15X+0.19$ |
| C22:6n-3 | $Y=0.00X+0.04$ | $Y=-0.01X+0.34$ | $Y=0.00X+0.20$ | $Y=0.00X-0.03$ | $Y=0.00X+0.00$ | $Y=0.00X+0.03$ | $Y=-0.01X+0.29$ | $Y=0.00X+0.05$ | $Y=0.00X+0.21$ | $Y=0.00X+0.08$ | $Y=0.06X+0.01$ | $Y=0.07X+0.03$ | $Y=0.17X+0.00$ | |

EP 2 702 395 B1

EP 2 702 395 B1

**Tableau 4: Exemple de matrice de corrélation ($r^2$) inter-acides gras (en mg/100g) obtenue sur la côte de porc. En italique, les relations significatives au seuil de 0.001%.**

| | C18:3n-3 | AGS | AGM | AGPI | n-6 | n-3 | C16:0 | C16:1n-7 | C18:1n-9c | C18:2n-6c | C20:4n-6 | C20:5n-3 | C22:5n-3 | C22:6n-3 |
|---|---|---|---|---|---|---|---|---|---|---|---|---|---|---|
| **C18:3n-3** | $r^2$=1.00 | *$r^2$=0.21* | *$r^2$=0.13* | *$r^2$=0.70* | *$r^2$=0.22* | *$r^2$=0.95* | *$r^2$=0.15* | $r^2$=0.00 | *$r^2$=0.12* | *$r^2$=0.18* | $r^2$=0.02 | *$r^2$=0.30* | *$r^3$=0.71* | $r^2$=0.02 |
| **AGS** | *$r^2$=0.21* | $r^2$=1.00 | *$r^2$=0.95* | *$r^2$=0.52* | *$r^2$=0.79* | *$r^2$=0.22* | *$r^2$=0.98* | *$r^2$=0.51* | *$r^2$=0.93* | *$r^2$=0.78* | $r^2$=0.04 | $r^2$=0.04 | *$r^2$=0.15* | $r^2$=0.00 |
| **AGM** | *$r^2$=0.13* | *$r^2$=0.95* | $r^2$=1.00 | *$r^2$=0.42* | *$r^2$=0.74* | *$r^2$=0.14* | *$r^2$=0.96* | *$r^2$=0.57* | *$r^2$=0.99* | *$r^2$=0.76* | $r^2$=0.03 | $r^2$=0.02 | *$r^2$=0.10* | $r^2$=0.00 |
| **AGPI** | *$r^2$=0.70* | *$r^2$=0.52* | *$r^2$=0.42* | $r^2$=1.00 | *$r^2$=0.67* | *$r^2$=0.80* | *$r^2$=0.42* | *$r^2$=0.07* | *$r^2$=0.38* | *$r^2$=0.52* | *$r^2$=0.22* | *$r^2$=0.42* | *$r^2$=0.57* | *$r^2$=0.08* |
| **n-6** | *$r^2$=0.22* | *$r^2$=0.79* | *$r^2$=0.74* | *$r^2$=0.6T* | $r^2$=1.00 | *$r^2$=0.25* | *$r^2$=0.76* | *$r^2$=0.42* | *$r^2$=0.73* | *$r^2$=0.94* | *$r^2$=0.14* | *$r^2$=0.09* | *$r^2$=0.16* | $r^2$=0.01 |
| **n-3** | *$r^2$=0.95* | *$r^2$=0.22* | *$r^2$=0.14* | *$r^2$=0.80* | *$r^2$=0.25* | $r^2$=1.00 | *$r^2$=0.14* | $r^2$=0.00 | *$r^2$=0.12* | *$r^2$=0.17* | *$r^2$=0.10* | *$r^2$=0.48* | *$r^2$=0.73* | *$r^2$=0.06* |
| **C16:0** | *$r^2$=0.15* | *$r^2$=0.98* | *$r^2$=0.96* | *$r^2$=0.42* | *$r^2$=0.76* | *$r^2$=0.14* | $r^2$=1.00 | *$r^2$=0.62* | *$r^2$=0.96* | *$r^2$=0.79* | $r^2$=0.02 | $r^2$=0.01 | *$r^2$=0.10* | $r^2$=0.00 |
| **C16:1n-7** | $r^2$=0.00 | *$r^2$=0.51* | *$r^2$=0.57* | *$r^2$=0.07* | *$r^1$=0.42* | $r^2$=0.00 | *$r^2$=0.62* | $r^2$=1.00 | *$r^2$=0.60* | *$r^2$=0.50* | $r^2$=0.00 | $r^2$=0.03 | $r^2$=0.00 | $r^2$=0.00 |
| **C18:1n-9c** | *$r^2$=0.12* | *$r^2$=0.93* | *$r^2$=0.99* | *$r^2$=0.38* | *$r^2$=0.73* | *$r^2$=0.12* | *$r^2$=0.96* | *$r^2$=0.60* | $r^2$=1.00 | *$r^2$=0.77* | $r^2$=0.02 | $r^2$=0.01 | *$r^2$=0.08* | $r^2$=0.00 |
| **C18:2n-6c** | *$r^2$=0.18* | *$r^2$=0.78* | *$r^2$=0.76* | *$r^2$=0.52* | *$r^2$=0.94* | *$r^2$=0.17* | *$r^2$=0.79* | *$r^2$=0.50* | *$r^2$=0.77* | $r^2$=1.00 | $r^2$=0.02 | $r^2$=0.01 | *$r^2$=0.11* | $r^2$=0.00 |
| **C20:4n-6** | $r^2$=0.02 | $r^2$=0.04 | $r^2$=0.03 | *$r^2$=0.22* | *$r^2$=0.14* | *$r^2$=0.10* | $r^2$=0.02 | $r^2$=0.00 | $r^2$=0.02 | $r^2$=0.02 | $r^2$=1.00 | *$r^2$=0.41* | $r^2$=0.06 | *$r^2$=0.31* |
| **C20:5n-3** | *$r^2$=0.30* | $r^2$=0.04 | $r^2$=0.02 | *$r^2$=0.42* | *$r^2$=0.09* | *$r^2$=0.48* | $r^2$=0.01 | $r^2$=0.03 | $r^2$=0.01 | $r^2$=0.01 | *$r^2$=0.41* | $r^2$=1.00 | *$r^2$=0.34* | *$r^2$=0.19* |
| **C22:Sn-3** | *$r^2$=0.71* | *$r^2$=0.15* | *$r^2$=0.10* | *$r^2$=0.57* | *$r^2$=0.16* | *$r^2$=0.73* | *$r^2$=0.10* | $r^2$=0.00 | *$r^2$=0.08* | *$r^2$=0.11* | $r^2$=0.06 | *$r^2$=0.34* | $r^2$=1.00 | *$r^2$=0.19* |
| **C22:6n-3** | $r^2$=0.02 | $r^2$=0.00 | $r^2$=0.00 | *$r^2$=0.08* | $r^2$=0.01 | $r^2$=0.06 | $r^2$=0.00 | $r^2$=0.00 | $r^2$=0.00 | $r^2$=0.00 | *$r^2$=0.31* | *$r^2$=0.19* | *$r^2$=0.19* | $r^2$=1.00 |

**Tableau 5: Exemple de matrice de corrélation ($r^2$) inter-acides gras (en % des acides gras totaux) obtenue sur sur la côte de porc. En italique, les relations significatives au seuil de 0.0001%.**

| X \ Y | C18:3n-3 | AGS | AGM | AGPI | n-6 | n-3 | C16:0 | C16:1n-7 | C18:1n-9c | C18:2n-6c | C20:4n-6 | C20:5n-3 | C22:5n-3 | C22:6n-3 |
|---|---|---|---|---|---|---|---|---|---|---|---|---|---|---|
| C18:3n-3 | | Y= 0.11X - 35.78 | Y= 0.08X + 34.30 | Y= 0.36X - 222.72 | Y= 0.42X - 96.85 | Y= 0.71X + 2.94 | Y= 0.16X + 19.41 | Y= -0.20X + 369.81 | Y= 0.09X + 53.00 | Y= 0.42X - 53.03 | Y= 1.11X + 281.35 | Y= 6.27X + 203.11 | Y= 21.12X - 91.08 | Y= 8.45X + 307.90 |
| AGS | Y= 1.88X + 2772.45 | | Y= 0.93X + 52.58 | Y= 1.28X + 1397.77 | Y= 3.34X - 31.86 | Y= 1.41X + 2745.86 | Y= 1.69X - 13.16 | Y= 15.63X + 971.69 | Y= 1.05X + 67.76 | Y= 3.60X + 24.54 | Y= 6.28X + 3085.24 | Y= 9.05X + 3214.06 | Y= 39.66X + 2602.89 | Y= -0.59X + 3411.58 |
| AGM | Y= 1.58X + 3064.21 | Y= 1.02X + 128.29 | | Y= 1.20X + 1709.57 | Y= 3.39X + 105.13 | Y= 1.19X + 3037.65 | Y= 1.74X + 59.78 | Y= 17.31X + 899.79 | Y= 1.13X + 2.49 | Y= 3.70X + 124.46 | Y= 5.73X + 3303.84 | Y= 7.03X + 3447.90 | Y= 34.27X + 2902.54 | Y= -7.71X + 3627.51 |
| AGPI | Y= 1.96X + 914.34 | Y= 0.41X + 181.80 | Y= 0.35X + 309.35 | | Y= 1.74X - 220.56 | Y= 1.53X + 855.11 | Y= 0.63X + 306.75 | Y= 3.19X + 1078.97 | Y= 0.38X + 365.72 | Y= 1.66X + 19.49 | Y= 8.65X + 1130.30 | Y= 17.19X + 1205.54 | Y= 44.22X + 677.26 | Y= 43.24X + 1420.05 |
| n-6 | Y= 0.51X + 857.61 | Y= 0.24X + 228.66 | Y= 0.22X + 243.11 | Y= 0.38X + 426.48 | | Y= 0.40X + 840.91 | Y= 0.39X + 233.38 | Y= 3.78X + 441.36 | Y= 0.25X + 245.53 | Y= 1.05X + 46.98 | Y= 3.20X + 864.98 | Y= 3.66X + 951.21 | Y= 10.94X + 807.63 | Y= 6.87X + 1005.30 |
| n-3 | Y= 1.33X + 20.84 | Y= 0.15X - 52.08 | Y= 0.12X + 42.67 | Y= 0.52X - 349.35 | Y= 0.62X - 169.61 | | Y= 0.21X + 40.52 | Y= -0.49X + 547.20 | Y= 0.12X + 82.45 | Y= 0.55X - 46.97 | Y= 3.40X + 295.41 | Y= 10.78X + 238.31 | Y= 29.36X - 126.02 | Y= 21.26X + 394.18 |
| C16:0 | Y= 0.93X + 1715.21 | Y= 0.58X + 46.20 | Y= 0.55X + 45.91 | Y= 0.67X + 968.44 | Y= 1.92X + 47.13 | Y= 0.67X + 1713.43 | | Y= 10.10X + 454.35 | Y= 0.62X + 43.45 | Y= 2.13X + 29.61 | Y= 2.39X + 1906.85 | Y= 2.93X + 1966.70 | Y= 18.85X + 1646.57 | Y= -9.50X + 2064.38 |
| C16:1n-7 | Y= -0.01X + 158.39 | Y= 0.03X + 44.56 | Y= 0.03X + 36.66 | Y= 0.02X + 123.15 | Y= 0.11X + 40.43 | Y= -0.01X + 160.36 | Y= 0.06X + 31.74 | | Y= 0.04X + 33.05 | Y= 0.13X + 32.27 | Y= 0.10X + 150.75 | Y= -0.36X + 163.73 | Y= -0.20X + 160.10 | Y= -0.68X + 158.42 |
| C18:1n-9c | Y= 1.31X + 2746.76 | Y= 0.89X + 148.11 | Y= 0.88X + 23.60 | Y= 1.01X + 1599.74 | Y= 2.98X + 123.38 | Y= 0.95X + 2740.33 | Y= 1.54X + 70.15 | Y= 15.69X + 741.45 | | Y= 3.30X + 89.01 | Y= 3.78X + 2993.88 | Y= 3.94X + 3103.76 | Y= 26.89X + 2642.13 | Y= -16.84X + 3249.89 |
| C18:2n-6c | Y= 0.43X + 793.39 | Y= 0.22X + 197.44 | Y= 0.20X + 202.77 | Y= 0.31X + 447.15 | Y= 0.90X + 14.08 | Y= 0.31X + 795.42 | Y= 0.37X + 182.90 | Y= 3.80X + 346.11 | Y= 0.23X + 192.68 | | Y= 1.20X + 877.32 | Y= 1.29X + 911.54 | Y= 8.47X + 767.23 | Y= -2.67X + 949.13 |
| C20:4n-6 | Y= 0.02X + 45.57 | Y= 0.01X + 31.45 | Y= 0.00X + 33.83 | Y= 0.03X + 11.57 | Y= 0.04X + 7.50 | Y= 0.03X + 37.76 | Y= 0.01X + 38.41 | Y= 0.05X + 44.55 | Y= 0.00X + 38.29 | Y= 0.02X + 33.99 | | Y= 0.92X + 31.68 | Y= 0.75X + 36.27 | Y= 4.53X + 35.23 |
| C20:5n-3 | Y= 0.05X + 5.17 | Y= 0.00X + 7.67 | Y= 0.00X + 11.14 | Y= 0.02X - 16.55 | Y= 0.02X - 2.57 | Y= 0.04X + 0.55 | Y= 0.00X + 13.81 | Y= -0.08X + 33.73 | Y= 0.00X + 14.92 | Y= 0.01X + 12.51 | Y= 0.44X - 1.25 | | Y= 1.28X - 4.40 | Y= 2.44X + 12.76 |
| C22:5n-3 | Y= 0.03X + 8.96 | Y= 0.00X + 7.79 | Y= 0.00X + 9.85 | Y= 0.01X + 0.15 | Y= 0.01X + 5.47 | Y= 0.01X + 8.55 | Y= 0.03X + 10.04 | Y= 0.01X + 21.75 | Y= -0.01X + 10.99 | Y= 0.00X + 8.11 | Y= 0.01X + 16.50 | Y= 0.07X + 14.66 | | Y= 1.10X + 16.35 |
| C22:6n-3 | Y= 0.00X + 2.93 | Y= 0.00X + 3.65 | Y= 0.00X + 3.98 | Y= 0.00X + 0.61 | Y= 0.00X + 2.20 | Y= 0.00X + 2.32 | Y= 0.00X + 4.41 | Y= 0.00X + 4.34 | Y= 0.00X + 4.52 | Y= 0.00X + 4.21 | Y= 0.07X + 0.10 | Y= 0.08X + 1.97 | Y= 0.17X + 0.20 | |

**Tableau 6 : Matrice d'équation de prédiction inter-acides gras (en % des acides gras totaux) obtenus sur le *Long dorsal* du jeune bovin (n=110). Equation du type Y = aX+b. En italique, les relations significatives au seuil de 0.0001%.**

| | AGS | AGMI | AGPI | AGPI n-3 | AGPIn-6 | C 16:0 | C 16:1 | C 18:1 et isomere | C 18:2 n-6 | C 18:3 n-3 | C 20:4 n-6 | C 20:5 n-3 | C 22:5 n-3 | C 22:6 n-3 |
|---|---|---|---|---|---|---|---|---|---|---|---|---|---|---|
| AGS | 1 | *-0,46* | *-0,56* | -0,19 | *-0,59* | *0,49* | -0,23 | *-0,46* | *-0, 63* | -0,09 | -0,33 | -0,17 | -0,19 | -0,39 |
| AGMI | *-0,46* | 1 | *-0,48* | *-0,63* | *-0,41* | 0,36 | *0,75* | *0,99* | *-0,37* | *-0,53* | *-0,59* | *-0,59* | *-0,64* | -0,52 |
| AGPI | *-0,56* | *-0,48* | 1 | *0,77* | *0,97* | *-0,82* | *-0,47* | *-0,47* | *0,96* | *0,59* | *0,87* | *0,68* | *0,79* | *0,76* |
| AGPI n-3 | -0,19 | *-0,63* | *0,77* | 1 | *0,61* | *-0,59* | *-0,51* | *-0,62* | *0,60* | *0,92* | *0,67* | *0,73* | *0,83* | *0,70* |
| AGPIn-6 | *-0,59* | *-0,41* | *0,97* | *0,61* | 1 | *-0,77* | *-0,40* | *-0,41* | *1,00* | *0,41* | *0,89* | *0,60* | *0,70* | *0,76* |
| C 16:0 | *0,49* | 0,36 | *-0,82* | *-0,59* | *-0,77* | 1 | *0,65* | 0,29 | *-0,79* | *-0,46* | *-0,64* | *-0,54* | *-0,59* | *-0,63* |
| C 16:1 | -0,23 | *0,75* | *-0,47* | *-0,51* | *-0,40* | *0,65* | 1 | *0,65* | *-0,38* | *-0,43* | *-0,47* | *-0,54* | *-0,54* | -0,52 |
| C 18:1 et isomere | *-0,46* | *0,99* | *-0,47* | *-0,62* | *-0,41* | 0,29 | *0,65* | 1 | *-0,37* | *-0,53* | *-0,59* | *-0,56* | *-0,63* | -0,49 |
| C 18:2 n-6 | *-0,63* | *-0,37* | *0,96* | *0,60* | *1,00* | *-0,79* | *-0,38* | *-0,37* | 1 | *0,40* | *0,84* | *0,56* | *0,66* | *0,75* |
| C 18:3 n-3 | -0,09 | *-0,53* | *0,59* | *0,92* | *0,41* | *-0,46* | *-0,43* | *-0,53* | *0,40* | 1 | *0,43* | *0,46* | *0,59* | *0,53* |
| C 20:4 n-6 | -0,33 | *-0,59* | *0,87* | *0,67* | *0,89* | *-0,64* | *-0,47* | *-0,59* | *0,84* | *0,43* | 1 | *0,76* | *0,85* | *0,73* |
| C 20:5 n-3 | -0,17 | *-0,59* | *0,68* | *0,73* | *0,60* | *-0,54* | *-0,54* | *-0,56* | *0,56* | *0,46* | *0,76* | 1 | *0,92* | *0,85* |
| C 22:5 n-3 | -0,19 | *-0,64* | *0,79* | *0,83* | *0,70* | *-0,59* | *-0,54* | *-0,63* | *0,66* | *0,59* | *0,85* | *0,92* | 1 | *0,81* |
| C 22:6 n-3 | -0,39 | -0,52 | *0,76* | *0,70* | *0,76* | *-0,63* | -0,52 | -0,49 | *0,75* | 0,53 | *0,73* | *0,85* | *0,81* | 1 |

**Tableau 7: Exemple de matrice de corrélation ($r^2$) inter-acides gras (en mg/100g) obtenue sur le *Long dorsal* du jeune bovin (n=110).
En italique, les relations significatives au seuil de 0.001%.**

| | AGS | AGMI | AGPI | AGPI n-3 | AGPI n-6 | C 16:0 | C 16:1 | C 18:1 et isomere | C 18:2 n-6 | C 18:3 n-3 | C 20:4 n-6 | C 20:5 n-3 | C 22:5 n-3 | C 22:6 n-3 |
|---|---|---|---|---|---|---|---|---|---|---|---|---|---|---|
| **AGS** | | *AGMI = 61.688 - .4352 * AGS* | *AGPI = 38.116 - .5568 * AGS* | AGPI n-3 = 3.7188 - .0425 * AGS | *AGPIn-6 = 30.191 - .4593 * AGS* | *C 16:0 = 5.9553 + .33753 * AGS* | C 16:1 = 3.9942 - .0328 * AGS | *C 18:1et isomere = 54.591 - .3669 * AGS* | *C 18:2 n-6 = 26.063 - .4005 * AGS* | C 18:3 n-3 = 1.6538 - .0142 * AGS | C 20:4 n-8 = 2.5465 - .0366 * AGS | C 20:5 n-3 = .45689 - .0056 * AGS | C 22:5 n-3 = .77009 - .0098 * AGS | C 22:6 n-3 = .50888 - .0066 * AGS |
| **AGMI** | *AGMI = 61.688 - .4352 * AGS* | | *AGPI = 29.422 - .4968 * AGMI* | AGPI n-3 = 7.5146 - .1507 * AGMI | *AGPIn-6 = 20.023 - .3340 * AGMI* | C 16:0 = 12.847 + .26007 * AGMI | *C 16:1 = - 2.133 + .11278 * AGMI* | *C 18:1et isomere = 2.8012 + .83763 * AGMI* | *C 18:2 n-6 = 15.497 - .2482 * AGMI* | *C 18:3 n-3 = 4.2578 - .0841 * AGMI* | *C 20:4 n-6 = 3.4083 - .0689 * AGMI* | *C 20:5 n-3 = 1.0393 - .0221 * AGMI* | *C 22:5 n-3 = 1.6315 - .0343 * AGMI* | C 22:6 n-3 = .58860 - .0113 * AGMI |
| **AGPI** | *AGPI = 38.116 - .5568 * AGS* | *AGPI = 29.422 - .4968 * AGMI* | | *AGPI n-3 = - .1823 + .17759 * AGPI* | *AGPIn-6 = - .5835 + .75682 * AGPI* | *C 16:0 = 28.689 - .5683 * AGPI* | *C 16:1 = 2.9861 - .0675 * AGPI* | *C 18:1et isomere = 39.631 - .3793 * AGPI* | *C 18:2 n-6 = - .3919 + .62113 * AGPI* | *C 18:3 n-3 = .05291 + .08978 * AGPI* | *C 20:4 n-6 = - .2759 + .09811 * AGPI* | *C 20:5 n-3 = - .0628 + .02346 * AGPI* | *C 22:5 n-3 = - .1220 + .04045 * AGPI* | *C 22:6 n-3 = .01502 + .01267 * AGPI* |
| **AGPI n-3** | AGPI n-3 = 3.7188 - .0425 * AGS | *AGPI n-3 = 7.5146 - .1507 * AGMI* | *AGPI n-3 = - .1823 + .17759 * AGPI* | | *AGPIn-6 = 3.5836 + 2.0807 * AGPI n-3* | *C 16:0 = 25.907 - 1.786 * AGPI n-3* | *C 16:1 = 2.8247 - .3206 * AGPI n-3* | *C 18:1et isomere = 39.330 - 2.193 * AGPI n-3* | *C 18:2 n-6 = 3.0790 + 1.6749 * AGPI n-3* | *C 18:3 n-3 = - .0202 + .61183 * AGPI n-3* | *C 20:4 n-6 = .17905 + .32459 * AGPI n-3* | *C 20:5 n-3 = - .0017 + .10842 * AGPI n-3* | *C 22:5 n-3 = .0144 + .18530 * AGPI n-3* | *C 22:6 n-3 = .07530 + .04432 * AGPI n-3* |
| **AGPI n-6** | *AGPIn-6 = 30.191 - .4593 * AGS* | *AGPIn-6 = 20.023 - .3340 * AGMI* | *AGPIn-6 = - .5835 + .75682 * AGPI* | *AGPIn-6 = 3.5836 + 2.0807 * AGPI n-3* | | *C 16:0 = 27.806 - .6856 * AGPIn-6* | *C 16:1 = 2.8291 - .0737 * AGPIn-6* | *C 18:1et isomere = 38.789 - .4206 * AGPIn-6* | *C 18:2 n-6 = .10273 + .81840 * AGPIn-6* | *C 18:3 n-3 = .39215 + .07902 * AGPIn-6* | *C 20:4 n-6 = - .1830 + .12711 * AGPIn-6* | *C 20:5 n-3 = - .0103 + .02610 * AGPIn-6* | *C 22:5 n-3 = - .0390 + .04588 * AGPIn-6* | *C 22:6 n-3 = .01777 + .01791 * AGPIn-6* |
| **C 16:0** | *C 16:0 = 5.9553 + .33753 * AGS* | C 16:0 = 12.847 + .26007 * AGMI | *C 16:0 = 28.689 - .5683 * AGPI* | *C 16:0 = 25.907 - 1.786 * AGPI n-3* | *C 16:0 = 27.806 - .6856 * AGPIn-6* | | *C 16:1 = - .8025 + .13527 * C 16:0* | C 18:1et isomere = 28.060 + .33988 * C 16:0 | *C 18:2 n-6 = 22.598 - .7313 * C 16:0* | *C 18:3 n-3 = 3.2503 - .1003 * C 16:0* | *C 20:4 n-6 = 3.0698 - .1032 * C 16:0* | *C 20:5 n-3 = .76707 - .0258 * C 16:0* | *C 22:5 n-3 = 1.2798 - .0435 * C 16:0* | *C 22:6 n-3 = .55812 - .0179 * C 16:0* |
| **C 16:1** | C 16:1 = 3.9942 - .0328 * AGS | *C 16:1 = - 2.133 + .11278 * AGMI* | *C 16:1 = 2.9861 - .0675 * AGPI* | *C 16:1 = 2.8247 - .3206 * AGPI n-3* | *C 16:1 = 2.8291 - .0737 * AGPIn-6* | *C 16:1 = - .8025 + .13527 * C 16:0* | | *C 18:1et isomere = 27.437 + 3.6473 * C 16:1* | *C 18:2 n-6 = 9.6419 - 1.702 * C 16:1* | *C 18:3 n-3 = 1.9750 - .4488 * C 16:1* | *C 20:4 n-6 = 1.5399 - .3681 * C 16:1* | *C 20:5 n-3 = .47196 - .1302 * C 16:1* | *C 22:5 n-3 = .71926 - .1916 * C 16:1* | C 22:6 n-3 = .32969 - .0825 * C 16:1 |
| **C 18:1 et isomere** | *C 18:1et isomere = 54.591 - .3669 * AGS* | *C 18:1et isomere = 2.8012 + .83763 * AGMI* | *C 18:1et isomere = 39.631 - .3793 * AGPI* | *C 18:1et isomere = 39.330 - 2.193 * AGPI n-3* | *C 18:1et isomere = 38.789 - .4206 * AGPIn-6* | C 18:1et isomere = 28.060 + .33988 * C 16:0 | *C 18:1et isomere = 27.437 + 3.6473 * C 16:1* | | *C 18:2 n-6 = 16.132 - .2909 * C 18:1et isomere* | *C 18:3 n-3 = 4.4848 - .0989 * C 18:1et isomere* | *C 20:4 n-6 = 3.6138 - .0816 * C 18:1et isomere* | *C 20:5 n-3 = 3.6138 - .0250 * C 18:1et isomere* | *C 22:5 n-3 = 1.7096 - .0400 * C 18:1et isomere* | C 22:6 n-3 = .60631 - .0129 * C 18:1et isomere |

EP 2 702 395 B1

## Suite tableau 7

| | | | | | | | | | | | | | |
|---|---|---|---|---|---|---|---|---|---|---|---|---|---|
| **C 18:2 n-6** | C 18:2 n-6 = 26.063 - .4005 * AGS | C 18:2 n-6 = 15.497 - .2482 * AGMI | C 18:2 n-6 = -.3919 + .62113 * AGPI | C 18:2 n-6 = 3.0790 + 1.6749 * AGPI n-3 | C 18:2 n-6 = .10273 + .81840 * AGPIn-6 | C 18:2 n-6 = 22.598 - .7313 * C 16:0 | C 18:2 n-6 = 9.6419 - 1.702 * C 16:1 | C 18:2 n-6 = 16.132 - .2909 * C 18:1et isomere | | C 18:3 n-3 = .38865 + .09542 * C 18:2 n-6 | C 20:4 n-6 = -.1511 + .14689 * C 18:2 n-6 | C 20:5 n-3 = -.4E-3 + .02968 * C 18:2 n-6 | C 22:5 n-3 = -.0251 + .05256 * C 18:2 n-6 | C 22:6 n-3 = .01749 + .02190 * C 18:2 n-6 |
| **C 18:3 n-3** | C 18:3 n-3 = 1.6538 - .0142 * AGS | C 18:3 n-3 = 4.2578 - .0841 * AGMI | C 18:3 n-3 = .05291 + .08978 * AGPI | C 18:3 n-3 = -.0202 + .61183 * AGPI n-3 | C 18:3 n-3 = .39215 + .07902 * AGPIn-6 | C 18:3 n-3 = 3.2503 - .1003 * C 16:0 | C 18:3 n-3 = 1.9750 - .4488 * C 16:1 | C 18:3 n-3 = 4.4848 - .0989 * C 18:1et isomere | C 18:3 n-3 = .38865 + .09542 * C 18:2 n-6 | | C 20:4 n-6 = .39008 + .31535 * C 18:3 n-3 | C 20:5 n-3 = .07463 + .10256 * C 18:3 n-3 | C 22:5 n-3 = .08704 + .20045 * C 18:3 n-3 | C 22:6 n-3 = .10920 + .05407 * C 18:3 n-3 |
| **C 20:4 n-6** | C 20:4 n-6 = 2.5465 - .0366 * AGS | C 20:4 n-6 = 3.4083 - .0689 * AGMI | C 20:4 n-6 = -.2759 + .09811 * AGPI | C 20:4 n-6 = .17905 + .32459 * AGPI n-3 | C 20:4 n-6 = -.1830 + .12711 * AGPIn-6 | C 20:4 n-6 = 3.0698 - .1032 * C 16:0 | C 20:4 n-6 = 1.5399 - .3681 * C 16:1 | C 20:4 n-6 = 3.6138 - .0816 * C 18:1et isomere | C 20:4 n-6 = -.1511 + .14689 * C 18:2 n-6 | C 20:4 n-6 = .39008 + .31535 * C 18:3 n-3 | | C 20:5 n-3 = .00860 + .23044 * C 20:4 n-6 | C 22:5 n-3 = .00734 + .38953 * C 20:4 n-6 | C 22:6 n-3 = .06345 + .10558 * C 20:4 n-6 |
| **C 20:5 n-3** | C 20:5 n-3 = .45689 - .0056 * AGS | C 20:5 n-3 = 1.0393 - .0221 * AGMI | C 20:5 n-3 = -.0628 + .02346 * AGPI | C 20:5 n-3 = -.0017 + .10842 * AGPI n-3 | C 20:5 n-3 = -.0103 + .02610 * AGPIn-6 | C 20:5 n-3 = .76707 - .0258 * C 16:0 | C 20:5 n-3 = .47196 - .1302 * C 16:1 | C 20:5 n-3 = 1.0626 - .0250 * C 18:1et isomere | C 20:5 n-3 = -.4E-3 + .02968 * C 18:2 n-6 | C 20:5 n-3 = .07463 + .10256 * C 18:3 n-3 | C 20:5 n-3 = .00860 + .23044 * C 20:4 n-6 | | C 22:5 n-3 = .04103 + 1.4086 * C 20:5 n-3 | C 22:6 n-3 = .05821 + .37095 * C 20:5 n-3 |
| **C 22:5 n-3** | C 22:5 n-3 = .77009 - .0098 * AGS | C 22:5 n-3 = 1.6315 - .0343 * AGMI | C 22:5 n-3 = -.1220 + .04045 * AGPI | C 22:5 n-3 = -.0144 + .18530 * AGPI n-3 | C 22:5 n-3 = -.0390 + .04586 * AGPIn-6 | C 22:5 n-3 = 1.2798 - .0435 * C 16:0 | C 22:5 n-3 = .71928 - .1916 * C 16:1 | C 22:5 n-3 = 1.7096 - .0400 * C 18:1et isomere | C 22:5 n-3 = -.0251 + .05256 * C 18:2 n-6 | C 22:5 n-3 = .08704 + .20045 * C 18:3 n-3 | C 22:5 n-3 = .00734 + .38953 * C 20:4 n-6 | C 22:5 n-3 = .04103 + 1.4086 * C 20:5 n-3 | | C 22:6 n-3 = .06052 + .23537 * C 22:5 n-3 |
| **C 22:6 n-3** | C 22:6 n-3 = .50888 - .0066 * AGS | C 22:6 n-3 = .58860 - .0113 * AGMI | C 22:6 n-3 = .01502 + .01267 * AGPI | C 22:6 n-3 = .07530 + .04432 * AGPI n-3 | C 22:6 n-3 = .01777 + .01791 * AGPIn-6 | C 22:6 n-3 = .55812 - .0179 * C 16:0 | C 22:6 n-3 = .32969 - .0825 * C 16:1 | C 22:6 n-3 = .60631 - .0129 * C 18:1et isomere | C 22:6 n-3 = .01749 + .02190 * C 18:2 n-6 | C 22:6 n-3 = .10920 + .05407 * C 18:3 n-3 | C 22:6 n-3 = .06345 + .10558 * C 20:4 n-6 | C 22:6 n-3 = .05821 + .37095 * C 20:5 n-3 | C 22:6 n-3 = .06052 + .23537 * C 22:5 n-3 |

**Tableau 8 : Matrice d'équation de prédiction inter-acides gras (en mg/100g) obtenus sur le *Long dorsal* du jeune bovin (n=110).**

**Equation du type Y = aX+b. En italique, les relations significatives au seuil de 0.001%.**

| | AGS | AGMI | AGPI | AGPI n-3 | C 16:0 | C 16:1 | C 18:1et isomere | C 18:2 n-6 | C 18:3 n-3 | C 20:4 n-6 | C 20:5 n-3 | C 22:5 n-3 | C 22:6 n-3 |
|---|---|---|---|---|---|---|---|---|---|---|---|---|---|
| AGS | *1,00* | *0,27* | 0,10 | 0,10 | *0,34* | *0,41* | *0,46* | 0,09 | 0,05 | -0,06 | 0,23 | 0,03 | 0,03 |
| AGMI | *0,27* | *1,00* | 0,02 | 0,01 | *0,32* | *0,48* | *0,38* | 0,03 | 0,01 | 0,06 | 0,16 | 0,08 | 0,10 |
| AGPI | 0,10 | 0,02 | *1,00* | 0,23 | -0,01 | 0,08 | 0,08 | *0,40* | *0,25* | *0,30* | *0,31* | *0,33* | 0,24 |
| AGPI n-3 | 0,10 | 0,01 | 0,23 | *1,00* | 0,03 | 0,01 | 0,15 | 0,21 | *0,69* | *0,31* | *0,45* | *0,37* | 0,37 |
| C 16:0 | *0,34* | *0,32* | -0,01 | 0,03 | *1,00* | *0,46* | *0,38* | 0,13 | -0,06 | 0,01 | 0,07 | 0,01 | -0,01 |
| C 16:1 | *0,41* | *0,48* | 0,08 | 0,01 | *0,46* | *1,00* | *0,50* | 0,12 | 0,09 | 0,04 | 0,09 | 0,03 | 0,11 |
| C 18:1et isomere | *0,46* | *0,38* | 0,08 | 0,15 | *0,38* | *0,50* | *1,00* | 0,10 | 0,09 | 0,04 | 0,21 | 0,04 | 0,27 |
| C 18:2 n-6 | 0,09 | 0,03 | *0,40* | 0,21 | 0,13 | 0,12 | 0,10 | *1,00* | 0,18 | *0,41* | *0,54* | *0,43* | *0,49* |
| C 18:3 n-3 | 0,05 | 0,01 | *0,25* | *0,69* | -0,06 | 0,09 | 0,09 | 0,18 | *1,00* | *0,30* | *0,35* | *0,32* | 0,23 |
| C 20:4 n-6 | -0,06 | 0,06 | *0,30* | *0,31* | 0,01 | 0,04 | 0,04 | *0,41* | *0,30* | *1,00* | *0,52* | *0,57* | 0,21 |
| C 20:5 n-3 | 0,23 | 0,16 | *0,31* | *0,45* | 0,07 | 0,09 | 0,21 | *0,54* | *0,35* | *0,52* | *1,00* | *0,64* | 0,43 |
| C 22:5 n-3 | 0,03 | 0,08 | *0,33* | *0,37* | 0,01 | 0,03 | 0,04 | *0,43* | *0,32* | *0,57* | *0,64* | *1,00* | 0,43 |
| C 22:6 n-3 | 0,03 | 0,10 | 0,24 | 0,37 | -0,01 | 0,11 | 0,27 | *0,49* | 0,23 | 0,21 | 0,43 | 0,43 | *1,00* |

EP 2 702 395 B1

**Tableau 9 : Matrice d'équation de prédiction inter-acides gras (en mg/100g) obtenus sur le *Long dorsal* du jeune bovin (n=110).**

**Equation du type Y = aX+b. En italique, les relations significatives au seuil de 0.001%.**

| | AGS | AGMI | AGPI | AGPI n-3 | C 16:0 | C 16:1 | C 18:1et isomere | C 18:2 n-6 | C 18:3 n-3 | C 20:4 n-6 | C 20:5 n-3 | C 22:5 n-3 | C 22:6 n-3 |
|---|---|---|---|---|---|---|---|---|---|---|---|---|---|
| AGS | | *AGMI = 471,37 + ,21747 * AGS* | AGPI = 159,22 + ,01271 * AGS | AGPI n-3 = 27,584 + ,00234 * AGS | *C 16:0 = 272,44 + ,15168 * AGS* | *C 16:1 = 28,865 + ,01996 * AGS* | *RAK C 18:1et isomere = 315,93 + ,34518 * AGS* | C 18:2 n-6 = 93,723 + ,00605 * AGS | C 18:3 n-3 = 17,531 + ,89E-3 * AGS | C 20:4 n-6 = 13,346 - ,6E-3 * AGS | C 20:5 n-3 = 2,9761 + ,65E-3 * AGS | C 22:5 n-3 = 5,4699 + ,13E-3 * AGS | C 22:6 n-3 = 3,3525 + ,30E-4 * AGS |
| AGMI | | | AGPI = 169,47 + ,00332 * AGMI | AGPI n-3 = 29,657 + ,35E-3 * AGMI | *C 16:0 = 298,48 + ,18006 * AGMI* | *C 16:1 = 28,174 + ,02970 * AGMI* | *RAK C 18:1et isomere = 411,06 + ,35747 * AGMI* | C 18:2 n-6 = 98,057 + ,00238 * AGMI | C 18:3 n-3 = 18,218 + ,27E-3 * AGMI | C 20:4 n-6 = 12,289 + ,72E-3 * AGMI | C 20:5 n-3 = 3,2144 + ,81E-3 * AGMI | C 22:5 n-3 = 5,3084 + ,41E-3 * AGMI | C 22:6 n-3 = 3,2747 + ,18E-3 * AGMI |
| AGPI | | | | AGPI n-3 = 22,576 + ,04261 * AGPI | C 16:0 = 429,03 - ,0412 * AGPI | C 16:1 = 43,083 + ,03176 * AGPI | RAK C 18:1et isomere = 576,89 + ,46170 * AGPI | *C 18:2 n-6 = 61,171 + ,22427 * AGPI* | *C 18:3 n-3 = 12,560 + ,03403 * AGPI* | *C 20:4 n-6 = 8,8619 + ,02273 * AGPI* | *C 20:5 n-3 = 2,3731 + ,00710 * AGPI* | *C 22:5 n-3 = 3,6084 + ,01161 * AGPI* | C 22:6 n-3 = 2,9667 + ,00199 * AGPI |
| AGPI n-3 | | | | | C 16:0 = 405,29 + ,55764 * AGPI n-3 | C 16:1 = 47,722 + ,02730 * AGPI n-3 | RAK C 18:1et isomere = 505,26 + 5,0488 * AGPI n-3 | C 18:2 n-6 = 80,767 + ,63301 * AGPI n-3 | *C 18:3 n-3 = 3,5358 + ,49742 * AGPI n-3* | *C 20:4 n-6 = 9,0108 + ,12560 * AGPI n-3* | *C 20:5 n-3 = 1,9774 + ,05308 * AGPI n-3* | *C 22:5 n-3 = 3,4934 + ,07018 * AGPI n-3* | C 22:6 n-3 = 2,8523 + ,01463 * AGPI n-3 |
| C 16:0 | | | | | | *C 16:1 = 27,236 + ,05048 * C 16:0* | *RAK C 18:1et isomere = 384,28 + ,64439 * C 16:0* | C 18:2 n-6 = 91,303 + ,01988 * C 16:0 | C 18:3 n-3 = 19,402 - ,0024 * C 16:0 | C 20:4 n-6 = 12,705 + ,14E-3 * C 16:0 | C 20:5 n-3 = 3,4050 + ,50E-3 * C 16:0 | C 22:5 n-3 = 5,5538 + ,96E-4 * C 16:0 | C 22:6 n-3 = 3,3924 - ,3E-4 * C 16:0 |
| C 16:1 | | | | | | | *RAK C 18:1et isomere = 279,96 + 7,7512 * C 16:1* | C 18:2 n-6 = 91,486 + ,16901 * C 16:1 | C 18:3 n-3 = 16,921 + ,03058 * C 16:1 | C 20:4 n-6 = 12,434 + ,00683 * C 16:1 | C 20:5 n-3 = 3,3464 + ,00537 * C 16:1 | C 22:5 n-3 = 5,4642 + ,00266 * C 16:1 | C 22:6 n-3 = 3,2399 + ,00348 * C 16:1 |
| C 18:1et isomere | | | | | | | | C 18:2 n-6 = 93,580 + ,00931 * RAK C 18:1et isomere | C 18:3 n-3 = 17,069 + ,00204 * RAK C 18:1et isomere | C 20:4 n-6 = 12,464 + ,46E-3 * RAK C 18:1et isomere | C 20:5 n-3 = 3,0858 + ,86E-3 * RAK C 18:1et isomere | C 22:5 n-3 = 5,4420 + ,23E-3 * RAK C 18:1et isomere | C 22:6 n-3 = 3,0871 + ,52E-3 * RAK C 18:1et isomere |
| C 18:2 n-6 | | | | | | | | | C 18:3 n-3 = 14,055 + ,04364 * C 18:2 n-6 | *C 20:4 n-6 = 7,3830 + ,05399 * C 18:2 n-6* | *C 20:5 n-3 = 1,4992 + ,02124 * C 18:2 n-6* | *C 22:5 n-3 = 2,9505 + ,02664 * C 18:2 n-6* | *C 22:6 n-3 = 1,7114 + ,01220 * C 18:2 n-6* |
| C 18:3 n-3 | | | | | | | | | | *C 20:4 n-6 = 9,6869 + ,16726 * C 18:3 n-3* | *C 20:5 n-3 = 2,5109 + ,05721 * C 18:3 n-3* | *C 22:5 n-3 = 4,0704 + ,08274 * C 18:3 n-3* | C 22:6 n-3 = 3,0243 + ,01617 * C 18:3 n-3 |
| C 20:4 n-6 | | | | | | | | | | | *C 20:5 n-3 = 1,5882 + ,15248 * C 20:4 n-6* | *C 22:5 n-3 = 2,2006 + ,26450 * C 20:4 n-6* | C 22:6 n-3 = 2,9619 + ,02275 * C 20:4 n-6 |
| C 20:5 n-3 | | | | | | | | | | | | *C 22:5 n-3 = 2,0630 + 1,0189 * C 20:5 n-3* | C 22:6 n-3 = 2,2670 + ,19607 * C 20:5 n-3 |
| C 22:5 n-3 | | | | | | | | | | | | | C 22:6 n-3 = 2,5655 + ,09662 * C 22:5 n-3 |
| C 22:6 n-3 | | | | | | | | | | | | | |

**Tableau 10: Exemple de corrélation et de prédiction inter-tissu chez le porc. Données obtenues pour l'acide alpha-linolénique (C18:3n-3, ALA) exprimé en % des acides gras totaux. Toutes les corrélations sont significatives au seuil de 0,001%.**

| | Bardière | Côte | Foie | Long Dorsal | Semi Membraneux | Plasma |
|---|---|---|---|---|---|---|
| **Bardière** | | n=49 ; $r^2$=0.98 Bardière = 1.28 x Cote crue + 0.04 | n=99 ; $r^2$=0.81 Bardière = 1.42 x Foie + 0.40 | n=129 ; $r^2$=0.93 Bardière = 2.23 x LD - 0.00 | n=52 ; $r^2$=0.97 Bardière = 1.98 x SM-0.14 | n=120 ; $r^2$=0.83 Bardière = 0.97 x plasma + 0.54 |
| **Côte** | n=49 ; $r^2$=0.98 Cote crue = 0.77 x Bardière +0.01 | | n=49 ; $r^2$=0.80 Cote crue = 1.02 x Foie + 0.31 | n=49 ; $r^2$=0.96 Cote crue = 1.83 x LD - 0.07 | n=49 ; $r^2$=0.97 Cote crue = 1.68 x SM - 0.24 | n=44 ; $r^2$=0.82 Cote crue = 0.58 x plasma +0.36 |
| **Foie** | n=99 ; $r^2$=0.81 Foie = 0.57 x Bardière + 0.10 | n=49 ; $r^2$=0.80 Foie = 0.78 x Cote crue + 0.05 | | n=99 ; $r^2$=0.82 Foie = 1.35 x LD - 0.01 | n=51 ; $r^2$=0.85 Foie = 1.23 xSM-0.11 | n=91 ; $r^2$=0.82 Foie = 0.6 x plasma + 0.30 |
| **Long Dorsal** | n=129 ; $r^2$=0.93 LD = 0.42 x Bardière + 0.10 | n=49 ; $r^2$=0.96 LD = 0.53 x Cote crue + 0.08 | n=99 ; $r^2$=0.82 LD = 0.61 x Foie + 0.23 | | n=52 ; $r^2$=0.97 LD = 0.87 x SM - 0.03 | n=121 ; $r^2$=0.79 LD = 0.49 x plasma + 0.21 |
| **Semi Membraneux** | n=52 ; $r^2$=0.97 SM = 0.49 x Bardière + 0.11 | n=49 ; $r^2$=0.97 SM = 0.58 x Cote crue + 0.17 | n=51 ; $r^2$=0.85 SM = 0.69 x Foie + 0.28 | n=52 ; $r^2$=0.97 SM = 1.12 x LD + 0.08 | | n=45 ; $r^2$=0.84 SM = 0.36 x plasma + 0.35 |

**Tableau 11: Exemple de corrélation et de prédiction inter-tissu chez le bovin. Données obtenues pour l'acide alpha-linolénique (C18:3n-3, ALA) exprimé en % des acides gras totaux et en mg/100g. Toutes les corrélations sont significatives au seuil de 0,001%.**

| | C18:3 % Long dorsal | C18:3 % Hampe | C18:3 % Bavette |
|---|---|---|---|
| C18:3 % Long dorsal | 1,00 | 0,57 | 0,61 |
| C18:3 % Hampe | 0,57 | 1,00 | 0,79 |
| C18:3 % Bavette | 0,61 | 0,79 | 1,00 |

| | C18:3 % Long dorsal | C18:3 % Hampe | C18:3 % Bavette |
|---|---|---|---|
| C18:3 % Long dorsal | | C18:3_%_H = ,24321 + ,51504 * | C183_%_B = ,21825 + ,61971 * |

|  |  | C183_%_LD | C183_%_LD |
|---|---|---|---|
| C18:3 % Hampe |  | ■ | C183_%_B = ,12810 + ,87619 * C183_%_H |
| C18:3 % Bavette |  |  | ■ |

|  | C18:3 mg Long dorsal | C18:3 mg Hampe | C18:3 mg Bavette |
|---|---|---|---|
| C18:3 mg Long dorsal | 1,00 | 0,76 | 0,57 |
| C18:3 mg Hampe | 0,76 | 1,00 | 0,72 |
| C18:3 mg Bavette | 0,57 | 0,72 | 1,00 |

|  | C18:3 mg Long dorsal | C18:3 mg Hampe | C18:3 mg Bavette |
|---|---|---|---|
| C18:3 mg Long dorsal | ■ | C183_mg_H = 4,2378 + 2,8594 * C183_mg_LD | C183_mg_B = 7,6913 + ,73464 * C183_mg_LD |
| C18:3 mg Hampe |  | ■ | C183_mg_B = 7,7733 + ,23975 * C183_mg_H |
| C18:3 mg Bavette |  |  | ■ |

**Tableau 12: Exemple de corrélation et de prédiction inter-tissu chez le bovin. Données obtenues pour l'acide palmitique (C16 :0) exprimé en % des acides gras totaux et en mg/100g. Toutes les corrélations sont significatives au seuil de 0,001%.**

|  | C16_% Long Dorsal | C16_% Hampe | C16_% Bavette |
|---|---|---|---|
| C16_% Long Dorsal | 1,00 | 0,84 | 0,89 |
| C16_% Hampe | 0,84 | 1,00 | 0,70 |
| C16_% Bavette | 0,89 | 0,70 | 1,00 |

|  | C16_% Long Dorsal | C16_% Hampe | C16_% Bavette |
|---|---|---|---|
| C16_% Long Dorsal |  | C16_%_H = 6,9466 + ,59343 * C16_%_LD | C16_%_B = 4,5996 + ,81918 * C16_%_LD |
| C16_% Hampe |  |  | C16_%_B = 4,1123 + ,93134 * C16_%_H |
| C16_% Bavette |  |  |  |

|  | C16_mg Long Dorsal | C16_mg Hampe | C16_mg Bavette |
|---|---|---|---|
| C16_mg Long Dorsal | 1,00 | 0,67 | 0,73 |
| C16_mg Hampe | 0,67 | 1,00 | 0,48 |

(suite)

|  | C16_mg Long Dorsal | C16_mg Hampe | C16_mg Bavette |
|---|---|---|---|
| C16_mg Bavette | 0,73 | 0,48 | 1,00 |

|  | C16_mg Long Dorsal | C16_mg Hampe | C16_mg Bavette |
|---|---|---|---|
| C16_mg Long Dorsal |  | *C16_g_H = 675,75 + 1,5175 * C16_g_LD* | *C16_g_B = 214,68 + ,97840 * C16_g_LD* |
| C16_mg Hampe |  |  | C16_g_B = 303,94 + ,28530 * C16_g_H |
| C16_mg Bavette |  |  |  |

**Revendications**

1. Méthode de détermination de la quantité d'au moins certains acides gras contenus dans différentes matières biologiques d'un même animal élevé pour sa production de viande, à partir d'une base de données dans laquelle on a déterminé préalablement, pour une matière de référence provenant du même type d'animal, les compositions ou profils en acides gras, déterminées par chromatographie en phase gazeuse, puis mesuré et enregistré les spectres d'absorption Infrarouge ou de diffusion Raman correspondants, méthode comprenant les étapes suivantes :

   a/ on détermine, par un modèle mathématique, pour un échantillon de cette matière de référence, une équation de calibration correspondant à la détermination, par une méthode spectroscopique, d'un acide gras ou d'une somme d'acides gras ;
   b/ on valide l'emploi de ladite équation de calibration de l'étape précédente, en vue de son utilisation avec de nouveaux échantillons à analyser, dès lors que pour une série d'échantillons dits « de validation », de même nature que la matière biologique de référence, le coefficient de corrélation $r^2$ entre la teneur en l'acide gras considéré ou la somme d'acides gras obtenue par chromatographie en phase gazeuse et par spectroscopie, est au moins égal à 0.7 ;
   c/ on soumet un nouvel échantillon à analyser, de même nature que la matière de référence, au rayonnement lumineux pour en obtenir le spectre d'absorption, et on utilise l'équation de calibration précédemment validée pour en déduire le profil, c'est à dire la teneur en acides gras dits « majeurs », à savoir ceux qui présentent un coefficient de corrélation $r^2$ au moins égal à 0.7 ;
   **caractérisé par le fait qu'**il comprend également au moins l'une des étapes suivantes :
   d/ on détermine le profil, c'est à dire la teneur en acides gras dits « mineurs », c'est à dire tous ceux dont le coefficient $r^2$ est inférieur à 0.7, à partir du profil en acides gras « majeurs » tel que déterminé à l'étape précédente, au moyen d'équations de prédiction statistiques présentant un coefficient de corrélation $r^2$ au moins supérieur au coefficient de corrélation $r^2$ déterminé entre les valeurs en acides gras dits « mineurs », mesuré par spectroscopie et les valeurs obtenues par chromatographie en phase gazeuse, ou au moyen d'équations de prédiction statistiques présentant un coefficient de corrélation au moins égal à 0.7, obtenu avec la méthode de chromatographie en phase gazeuse ;
   e/ on détermine la teneur en acides gras d'au moins une autre matière du même animal, à partir des profils obtenus aux étapes c/ et/ou d/, au moyen d'équations de prédiction statistiques présentant un coefficient de corrélation au moins égal à 0.7, obtenu avec la méthode de chromatographie en phase gazeuse.

2. Méthode selon la revendication 1, **caractérisé par le fait qu'**aux étapes b/, c/, d/ et e/, $r^2$ est au moins égal à 0.8, préférentiellement au moins égal à 0.9. l'une

3. Méthode selon l'une des revendications précédentes, **caractérisé par le fait que** ladite matière biologique est liquide.

4. Méthode selon la revendication 3, **caractérisé par le fait que** ladite matière biologique est du sang.

5. Méthode selon l'une des revendications 1 ou 2, **caractérisé par le fait que** ladite matière biologique est solide.

6. Méthode selon la revendication 5, **caractérisé par le fait que** la matière biologique est choisi parmi les poils, les écailles, les plumes, la peau, le gras, la viande, les abats.

**7.** Méthode selon l'une des revendications précédentes, **caractérisé par le fait que** ledit animal est choisi dans le groupe constitué des bovins, des ovins, de la volaille, des porcs, des lapins et des poissons.

**Patentansprüche**

**1.** Verfahren zur Bestimmung der Menge von zumindest einigen Fettsäuren, die in verschiedenen biologischen Materialien desselben Tiers, das zur Fleischproduktion aufgezogen wurde, enthalten sind, ausgehend von einer Datengrundlage, bei der zuvor für ein Referenzmaterial, welches von der gleichen Tierart stammt, die durch Gasphasenchromatographie ermittelten Fettsäure-Zusammensetzungen oder - Profile bestimmt wurden und dann die entsprechenden Infrarot- oder Raman-Streuung-Absorptionsspektren gemessen und aufgezeichnet wurden, wobei das Verfahren die folgenden Schritte umfasst:

a/ Aufstellen einer Kalibrierungsgleichung, in Übereinstimmung mit der Bestimmung einer Fettsäure oder einer Fettsäuremenge durch ein spektroskopisches Verfahren, für eine Probe des Referenzmaterials anhand eines mathematischen Modells,
b/ Validierung der Anwendung der Kalibrierungsgleichung aus dem vorherigen Schritt im Hinblick auf ihre Verwendung mit neuen zu analysierenden Proben, sofern für eine Reihe von sogenannten Proben "zur Validierung", die von der gleichen Beschaffenheit sind wie das biologische Referenzmaterial, der Korrelationskoeffizient $r^2$ zwischen dem angenommenen Fettsäuregehalt oder der durch Gasphasenchromatographie erhaltenen Fettsäuremenge und der durch Spektroskopie erhaltenen Fettsäuremenge wenigstens 0,7 beträgt;
c/ Aussetzen einer neuen zu analysierenden Probe, die von der gleichen Beschaffenheit ist wie das Referenzmaterial, einer Lichtstrahlung zum Erhalt des Absorptionsspektrums und Verwendung der zuvor validierten Kalibrierungsgleichung zum Ableiten des Profils, d.h., des Gehalts an sogenannten wichtigen" Fettsäuren, nämlich der, die einen Korrelationskoeffizienten $r^2$ von wenigstens 0,7 aufweisen;
**dadurch gekennzeichnet, dass** es außerdem wenigstens einen der folgenden Schritte umfasst:
d/ Bestimmung des Profils, d.h., des Gehalts an sogenannten "zweitrangigen" Fettsäuren, d.h., derjenigen Fettsäuren, deren Koeffizient $r^2$ unter 0,7 liegt, ausgehend von dem Profil der "wichtigen" Fettsäuren, wie im vorherigen Schritt bestimmt, anhand von statistischen Vorhersagegleichungen, die einen Korrelationskoeffizienten $r^2$ aufweisen, der zumindest größer ist als der Korrelationskoeffizient $r^2$, der zwischen den Werten der als "zweitrangig" bezeichneten Fettsäuren, gemessen durch Spektroskopie, und den Werten, die durch Gasphasenchromatographie erhalten wurden, bestimmt wurde, oder anhand von statistischen Vorhersagegleichungen, welche einen Korrelationskoeffizienten von wenigstens 0,7 aufweisen, der mit dem Gasphasenchromatgraphieverfahren erhalten wurde;
e/ Bestimmung des Gehalts an Fettsäuren von wenigstens einem weiteren Material desselben Tiers, ausgehend von den in den Schritten c/ und/oder d/ erhaltenen Profilen, anhand von statistischen Vorhersagegleichungen, welche einen Korrelationskoeffizienten von wenigstens 0,7 aufweisen, der mit dem Gasphasenchromatgraphieverfahren erhalten wurde.

**2.** Verfahren gemäß Anspruch 1, **dadurch gekennzeichnet, dass** in den Schritten b/, c/, d/ und e/ $r^2$ wenigstens 0,8, bevorzugt wenigstens 0,9, beträgt.

**3.** Verfahren gemäß einem der vorhergehenden Ansprüche, **dadurch gekennzeichnet, dass** das biologische Material flüssig ist.

**4.** Verfahren gemäß Anspruch 3, **dadurch gekennzeichnet, dass** das biologische Material Blut ist.

**5.** Verfahren gemäß einem der Ansprüche 1 oder 2, **dadurch gekennzeichnet, dass** das biologische Material fest ist.

**6.** Verfahren gemäß Anspruch 5, **dadurch gekennzeichnet, dass** das biologische Material aus Haaren, Schuppen, Federn, Haut, Fett, Fleisch und Innereien ausgewählt ist.

**7.** Verfahren gemäß einem der vorhergehenden Ansprüche, **dadurch gekennzeichnet, dass** das Tier aus der Gruppe bestehend aus Rindern, Schafen, Geflügel, Schweinen, Kaninchen und Fischen ausgewählt ist.

**Claims**

1. Method for determining the amount of at least some of the fatty acids contained in various biological materials of the same animal raised for meat production, from a database in which the fatty acid compositions or profiles have been determined beforehand, for a reference material coming from the same type of animal, determined by gas phase chromatography, then measuring and recording the corresponding infrared absorption spectra or corresponding Raman scattering, which method comprises the following steps:

   a/a calibration equation corresponding to the determination of a fatty acid or a sum of fatty acids by a spectroscopic method is determined for a sample of this reference material by a mathematic model;
   b/ the use of said calibration equation of the preceding step is validated, in view of its use with new samples to be analyzed, since for a series of so-called "validation" samples of the same nature as the biological reference material, the correlation coefficient $r^2$ between the fatty acid content considered or the sum of fatty acids obtained by gas phase chromatography and spectroscopy, is at least equal to 0.7;
   c/a new sample to be analyzed, of the same nature as the reference material, is subjected to light radiation to obtain the absorption spectrum, and the previously validated calibration equation is used to deduce the profile, i.e., the so called "major" fatty acid content, namely those with a correlation coefficient $r^2$ at least equal to 0.7;
   **characterized by** the fact that it also comprises at least one of the following steps:
   d/ the profile, i.e., the so-called "minor" fatty acid content, namely those whose coefficient $r^2$ is less than 0.7, is determined from the profile of "major" fatty acids as determined in the previous step, using statistical prediction equations with a correlation coefficient $r^2$ at least greater than correlation coefficient $r^2$ determined between the so-called "minor" fatty acids, measured by spectroscopy and the values obtained by gas phase chromatography, or using statistical prediction equations with a correlation coefficient at least equal to 0.7, obtained with the gas phase chromatography method;
   e/ the fatty acid content of at least one other material from the same animal is determined, from the profiles obtained in steps c/ and/or d/, by means of statistical prediction equations with a correlation coefficient at least equal to 0.7, obtained with the gas phase chromatography method.

2. Method according to claim 1, **characterized by** the fact that in steps b/, c/, d/ and e/, $r^2$ is at least equal to 0.8, preferably at least equal to 0.9.

3. Method according to one of the preceding claims, **characterized by** the fact that said biological material is fluid.

4. Method according to claim 3, **characterized by** the fact that said biological material is blood.

5. Method according to claim 1 or 2, **characterized by** the fact that said biological material is solid.

6. Method according to claim 5, **characterized by** the fact that: the biological material is chosen from among fur, scales, feathers, skin, fat, meat or offal.

7. Method according to one of the preceding claims, **characterized by** the fact that said animal is chosen in the group consisting of cattle, sheep, poultry, pigs, rabbits and fish.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

*   Rapid determination of the fatty acid profile in pork dry-cured sausages by NIR spectroscopy. **FERNAN-DEZ-CABANÁS V. M. et al.** FOOD CHEMISTRY. ELSEVIER LTD, 01 Janvier 2011, vol. 124